# EUROPEAN PATENT APPLICATION

(11) **EP 1 174 505 A1**
(43) Date of publication of application: **23.01.2002**
(21) Application number: 00202309.1
(22) Date of filing: 30.06.2000
(51) Int. Cl.: C12N 15/31, C07K 14/235, C07K 16/12, A61K 38/00, A61K 39/00, C12N 15/62, A61P 31/04

(54) **(Poly)peptides for the preparation of vaccines against Bordetella pertussis and/or Bordetella parapertussis, vaccines based upon such (poly)peptides, and antibodies against such peptides**

(71) Applicant: DE STAAT DER NEDERLANDEN VERTEGENWOORDIGD DOOR DE MINISTER VAN WELZIJN, VOLKSGEZONDHEID EN CULTUUR, NL-2280 HK Rijswijk (NL)
(72) Inventor: Mooi, Frederik Robert, 3961 JG Wijk bij Duurstede (NL)
(74) Representative: Jorritsma, Ruurd

(57) **Abstract**

The invention relates to a peptide comprising:
a) between 6 and 40 contiguous amino acid residues from the amino acid sequence of "region 1" of a *Bordetella pertussis* pertactin; and
b) a total of between 6 and 100, preferably between 6 and 50 amino acid residues; or an analog of such a peptide; optionally in the form of a salt acceptable for pharmaceutical and/or veterinary purposes.

The invention also relates to a polypeptide having the amino acid sequence of a pertactin from which, compared to the sequence of said pertactin:
a) the amino acid residues that form the so-called "region 1" have been at least partly, and preferably essentially completely removed; and/or from which,
b) the amino acid residues that form the so-called "region 2" have been at least partly, and preferably essentially completely removed.

The invention further relates to a pharmaceutical composition, and in particular a vaccine against (at least) *Bordetella pertussis* and/or *Bordetella parapertussis,* comprising the peptide and/or the polypeptide mentioned above.

Also, the invention comprises an antibody, generated/elicited against such a peptide and/or polypeptide, and to a pharmaceutical and/or veterinary composition comprising such an antibody.

## Description

The present invention relates to (poly)peptides that can be used for pharmaceutical and/or veterinary purposes, and in particular in the preparation of vaccines.

More in particular, the invention relates to (poly)peptides that can be used in the preparation of vaccines against *Bordetella pertussis* and/or *Bordetella parapertussis* (henceforth collectively referred to as "*Bordetella (para)pertussis*".)

The invention also relates to vaccines containing such (poly)peptides, and to the use of such peptides in the preparation of vaccines.

The invention further relates to antibodies generated against the aforementioned (poly)peptides, and to pharmaceutical compositions containing such antibodies.

In one specific embodiment of the invention, the (poly)peptide is a peptide derived from the so-called "region 1" of *Bordetella pertussis* pertactin, i.e. as further described hereinbelow.

In another specific embodiment of the invention, the polypeptide is a *Bordetella pertussis* pertactin from which (at least part of) said "region 1" and/or from which at least part of the so-called "region 2"has been removed.

Other aspects of the invention will become clear from the further description given hereinbelow.

Conventionally, vaccines against pertussis ("whooping-cough") have been based on whole cells of *Bordetella pertussis*. In recent years, besides these so-called "whole cell vaccines" or "WCV's", also vaccines have been developed that contain specific antigens derived from *Bordetella pertussis,* such as fimbriae, filamentous hemagglutinin, pertussis toxin and/or a surface-associated protein of *Bordetella pertussis* referred to as "pertactin". These so-called "acellular vaccines"or "ACVs" have now been introduced in several countries.

The sequence of *Bordetella pertussis* pertactin has been published (vide inter alia Charles et al., Proc. Natl. Acad. Sci. USA 1989 May; 86(10): 3554-8 and SEQ ID NO:6 below). Pertactin is a 69 kD protein comprising approximately 926 amino acid residues, which from X-ray data is known to form a helix with several protruding loops that contain sequence motifs associated with the biological activity of the protein (Emsley at al., Nature (1996) 381:90-92). These include a loop containing an Arg-Gly-Asp (RGD) tripeptide motif (a.a. 260-262) involved in adherence to host tissues and a loop with a (PQP)₅ motif near the carboxy- terminus containing the a major immunoprotective epitope (Charles et al., Eur. J. Immunol. (1991) 21:1147-1153.).

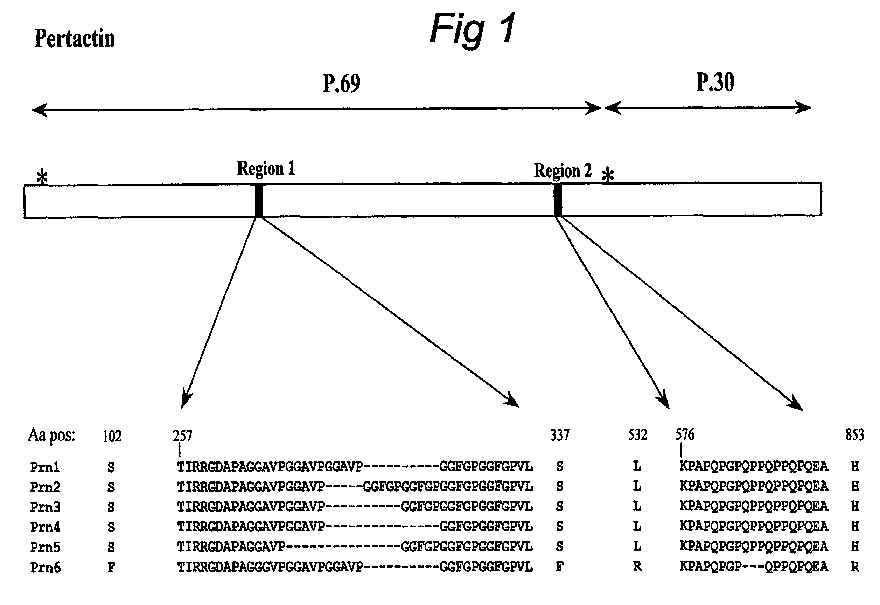

It is also known that pertactin is formed from a precursor, which is encoded by a gene referred to as *"prn".* This gene, which is schematically shown in Figure 1, comprises two regions, referred to as "region 1" and "region 2" respectively (vide also Mooi et al., Infect. Immun. (1998) 66:670-675). Of these, region 1 flanks the RGD motif and comprises a number of GGxxP repeats, whereas region 2 comprises the repeated PQP motif mentioned above.

Although WCV's have been very successful in the past and are still very effective in preventing *Bordetella pertussis* infections, there are signs that pertussis may be reemerging, also in Western countries with long traditions in pertussis vaccination, such as the United States, the Netherlands, Canada and Australia ( Mooi et al., Infect. Immun. (1999) 67:3133-3134). One explanation for this phenomenon has already been proposed by the present inventors: antigenic divergence between vaccine strains and circulating strains, in particular with respect to pertactin.

In support of this explanation, the present inventors have also shown that the *prn* gene that encodes the pertactin precursor is polymorphic. So far, up to 9 different pertactin types have been identified in *Bordetella pertussis* strains circulating in the world. It was also shown that variation between these pertactin types is mainly restricted to region 1, and consists of deletions or insertions in the repeat unit GGxxP of region 1. Reference is again made to Mooi et al. (1998); Mooi et al. (1999); and Mastrantonio et al. (Microbiology (1999) 145: 2069; all mentioned above.

Besides *Bordetella pertussis,* about 1 to 5 percent of pertussis cases is caused by (strains of) *Bordetella parapertussis*. In this respect, however, there is some evidence that ACV's based upon *Bordetella pertussis* antigens - including ACV's based upon intact *Bordetella pertussis* pertactin - may not (also) provide sufficient protection against (strains of) *Bordetella parapertussis*. For instance, tests in a mouse model have shown that immunization with intact *Bordetella pertussis* pertactin does provide protection against *Bordetella parapertussis* (Khelef et al., Infect. Immun. 1993; 61(2); 486-90).

Thus, it may be that the introduction of this novel generation of ACV's may in future lead to a relative increase in whooping cough cases that are caused by *Bordetella parapertussis*.

Thus, as the pertussis vaccines currently used may not be equally effective in providing protection against all these different strains of *Bordetella pertussis,* and may not be effective in providing protection against (strains of) *Bordetella parapertussis*, the development of novel vaccines against *Bordetella (para)pertussis*, and of antigens that may be used in the preparation of such vaccines, remains an ongoing concern, in order to ensure the long term efficacy of pertussis vaccines.

Accordingly, it is a general object of the invention to make available a vaccine that can be used to provide immunity against essentially all strains of *Bordetella pertussis,* or at least against essentially all strains of *Bordetella pertussis* that are currently known to circulate in the countries mentioned above, and in particular in Europe. Preferably, however, such a vaccine would also be able to provide protection against (all) other strains of *Bordetella pertussis,* including but not limited to those strains that are yet to be identified and/or that may arise in future. Most preferably, such a vaccine should also provide protection against (strains of) *Bordetella parapertussis*, or at least provide a degree of protection against (strains of) *Bordetella parapertussis* that is better than the protection provided by the current generation ACV's based upon *Bordetella pertussis* antigens, and in particular upon essentially intact *Bordetella pertussis* pertactin.

In particular, it is an object of the invention to provide a pharmaceutical and/or veterinairy component that upon administration to a human being (infant) or (other) mammal can provide protection against *Bordetella (para)pertussis,* e.g. by generating a protective (immune) response in said human being or mammal. As will be clear to the skilled person, such a component could be used as an antigen(ic) component in the preparation/formulation of vaccines against pertussis, both for human or veterinary application, including but not limited to combination vaccines known per se, such as "DTP" vaccines.

Surprisingly, it has now been found that the above objects can be achieved by the use of a peptide derived from "region 1" of *Bordetella pertussis* pertactin, and in particular by the use of a "peptide of the invention" as defined herein below.

*Inter alia*, this finding is particularly surprising because - whereas the use of an intact pertactin protein (e.g. as present on a *Bordetella pertussis* cell in an WCV or as used as an antigen in an ACV) in practice does not lead to a vaccine that is equally efficacious against the different strains of *Bordetella pertussis -* it has now been found that such cross-immunity can be obtained by using a vaccine based upon a (single) small protein derived from said pertactin. Also, it is expected that - despite the differences in the pertactine of *Bordetella pertussis* and the pertactine of *Bordetella parapertussis -* the vaccines of the invention will also provide at least some degree of protection against (strains of) *Bordetella parapertussis*, e.g. better than the protection provided by the current generation of ACV's.

This finding is even more surprising because - as mentioned above - these strains of *Bordetella pertussis* differ in that they have/express distinct pertactin types on their surface, and because between these distinct types, variation is essentially restricted to "region 1", i.e. the same amino acid sequence/epitope from which the peptide(s) used in the present invention are now derived.

It is conceivable that infection or vaccination mainly results in the induction of antibodies directed against conformational epitopes of pertactin. In this context it is significant that monoclonal antibodies directed against conformational epitopes in region 1 were type specific, whereas those that recognised a linear epitope bound to all *Bordetella pertussis* pertactin variants. Thus, to prevent cross-immunity, the bacteria may have evolved mechanisms to direct the immune response towards conformational epitopes. Extending this line of reasoning, it is plausible that the vaccination with a peptide derived from region 1 will induce cross-protective antibodies, as they will be directed against a linear epitope. It may also be that - in contrast to antibodies generated against conformational epitopes of *Bordetella pertussis* pertactin - such antibodies against a linear epitope of *Bordetella pertussis* pertactin can also bind to *Bordetella parapertussis* pertactin.

However, it should be noted that the present invention is not limited to any specific explanation of, nor to any mechanism for, the efficiacy of the peptides described hereinbelow, nor for the way in which these peptides provide their advantageous cross-immunity.

Thus, in a first aspect, the invention relates to a peptide that:
a) comprises between 6 and 40, and in particular between 10 and 15, contiguous amino acid residues from the amino acid sequence of "region 1" of a *Bordetella pertussis* pertactin; and that
b) comprises a total of between of between 6 and 100 amino acid residues, and in particular of between 6 and 50 amino acid residues, more in particular between 10 and 40 and amino acid residues.

In particular, the invention relates to a peptide that:
a) comprises between 6 and 36, and in particular between 10 and 15, contiguous amino acid residues from the amino acid sequence of SEQ ID NO: 1: TIRRGDAPAGGAVPGGAVPGGAVPGGFGPGGFGPVL (SEQ ID NO:1) in which the capital letters denote amino acid residues according to the standard "one letter protein" code; and that
b) comprises a total of between of between 6 and 100 amino acid residues, and in particular of between 6 and 50 amino acid residues, more in particular between 10 and 50 amino acid residues.

The peptide(s) mentioned above will also be referred to hereinbelow as "*peptide(s) of the invention*".

Usually, the peptides of the invention will consist essentially only of amino acid residues derived from/corresponding to the amino acid residues of region 1 of a Bordetella pertussis pertactin, and thus contain a total of between 6 and 40, preferably between 10 and 15 amino acid residues. However, as will be clear from the definitions given hereinabove and also from the further description hereinbelow, the presence of some additional amino acid residues is not excluded, e.g. to a total of 100 amino acid residues, preferably 50 amino acid residues.

These additional amino acid residues may for instance correspond to one or more amino acid residues of the sequence of pertactin that lie "outside" region 1. For instance, these may be amino acid residues that in the sequence of pertactin lie adjacent to (the sequence of) region 1, e.g. the first 20, and preferably 10, amino acid residues that lie directly preceding and/or directly following (the sequence of) region 1.

However, the invention is in its broadest sense is not limited to the further amino acid residues that may be present (i.e. additional to the amino acids of region 1); as long as the further requirements outlined herein are met. In addition, it should also be noted that - as outlined above - "analogs" of the peptides of the invention may be used, including but not limited to analogs into which one or more amino acid residues have been inserted, and or to which one or more amino acid residues have been added. Also, as described below, the peptides of the invention may be provided/used as a protein fusion with at least one amino acid sequence with which the peptide of the invention is not naturally associated (i.e. in (a) native pertactin).

The invention also relates to a pharmaceutical composition, and in particular to a vaccine, that comprises at least one peptide of the invention, optionally in combination with at least one pharmaceutically acceptable carrier, excipiens or adjuvans. Such a vaccine will also be referred to hereinbelow as a "*vaccine of the invention".*

In another aspect, the invention relates to the use of a peptide of the invention in the preparation of a vaccine, and in particular in the preparation of a vaccine against *Bordetella (para)pertussis* and optionally against one or more other infectious diseases of a human being, including but not limited to diphteria, tetanus, polio and/or *Haemophilus influenzae* b ("Hib").

The invention also relates to an antibody specific for, and/or generated against, a peptide of the invention, and to a pharmaceutical composition containing at least one such antibody, optionally in combination with at least one pharmaceutically acceptable carrier, excipiens or adjuvans.

In yet another aspect, the invention relates to a protein fusion comprising at least one peptide of the invention, and to a pharmaceutical composition that comprises at least one such protein fusion, optionally in combination with at least one pharmaceutically acceptable carrier, excipiens or adjuvans.

Further aspects and embodiments of the invention will become clear from the further description given hereinbelow.

Preferably, a peptide of the invention is such that, upon administration to a human being in a suitable manner and in a suitable amount (e.g. as outlined below), it is capable of generating/eliciting in said human being an immune response.

More preferably, a peptide of the invention is such that, upon administration to a human being in a suitable manner and in a suitable amount, it is capable of generating/eliciting in said human being a protective immune response, and in particular an immune response which protects said human being against infection by *Bordetella pertussis,* and preferably also against infection by *Bordetella parapertussis*.

Also, a peptide of the invention is preferably such that, upon administration to a human being in a suitable manner and in a suitable amount, it is capable of generating/eliciting in said human being a "detectable immune response", by which is generally meant an immune response that leads to at least one detectable biological change, and in particular to at least one detectable immunological change, in said human being. For instance, such a detectable immunonlogical change can involve the generation of antibodies against the peptide of the invention, against (essentially intact) pertactin, and/or against (whole cells of) *Bordetella (para)pertussis,* or any other detectable and/or measurable immune response.

Techniques for determining whether a peptide of the invention can provide such a "protective immune response" and/or such a "detectable immune response" will be clear to the skilled person, and may be essentially the same as, or may be essentially analogous to, the methods described in the Experimental Part below.

For instance, such a technique may involve immunizing a mouse or another suitable animal (mammal) with a peptide of the invention, and then determining whether antibodies are raised against said peptide, e.g. essentially as outlined in the Section 3C of the Experimental Part. Alternatively, an animal immunized with a peptide of the invention may be exposed to viable *Bordetella (para)pertussis* cells, after which the susceptibility to infection is compared to animals which have not been immunized, e.g. by means of determining the degree of colonization of the trachea and/or the lungs, e.g. essentially as outlined in Section 3D of the Experimental Part.

Particular preferred are those peptides of the invention which in a such test provide at least 10%, preferably at least 30%, more preferably at least 50%, and even more preferably at least 70%, of the protection provided by the peptide of SEQ ID NO:5 mentioned above, as may for instance be determined by the titers of antibodies raised (e.g. determined essentially as outlined in the Section 3C of the Experimental Part) and/or the degree of protection against infection provided (e.g. determined essentially as outlined in Section 3D of the Experimental Part).

Preferred peptides of the invention will generally contain one or more repeats GGFGP and/or one or more repeats GGAVP, in which the total number of such repeats will be between 2 and 10. For example, such preferred peptides of the invention include, but are not limited to:
- GGFGP-GGFGP (SEQ ID NO:2)
- GGAVP-GGAVP (SEQ ID NO:3)
- GGAVP-GGAVP-GGFGP-GGFGP (SEQ ID NO:4)
in which the capital letters again denote amino acid residues according to the standard "one letter protein" code.

Most preferred in the invention is the peptide of SEQ ID NO:5 GGAVPGGFGPGGFGP (SEQ ID NO:5) in which the capital letters again denote amino acid residues according to the standard "one letter protein" code.

Besides the above peptides of the invention, the invention also comprises (the use of) analogs of such peptides, by which is generally meant a peptide of the invention as defined above in which at most 8, preferably at most 4, more preferably at most two, and even more preferably only one amino acid residue has been deleted, replaced, added and/or substituted (the latter preferably as a "conservative" amino acid substitution, as will be known to the skilled person).

Preferably, such an analog is such that, upon administration to a human being in a suitable manner and in a suitable amount, it is capable of generating/eliciting in said human being an immune response.

More preferably, such an analog is such that, upon administration to a human being in a suitable manner and in a suitable amount, it is capable of generating/eliciting in said human being a protective immune response, and in particular an immune response which protects said human being against infection by *Bordetella pertussis,* and preferably also against infection by *Bordetella parapertussis*, as may be determined using one or more of the techniques referred to above.

Also, such an analog is preferably such that, upon administration to a human being in a suitable manner and in a suitable amount, it is capable of generating/eliciting in said human being a "detectable immune response", which for an analog may also include the generation against antibodies against the specific analog used, and as again may be determined using one or more of the techniques referred to above.

Particularly preferred are analogs of the peferred peptides of the invention as defined above. Especially preferred are analogs of the peptide of SEQ ID NO:5.

In the further description below, the analogs defined above should be considered as encompassed within the term "peptides of the invention", unless indicated otherwise. Also, the peptides of the invention and/or analogs thereof may be (used) in the form of a pharmaceutically acceptable salt, and such salts should also be considered as encompassed within the term "peptides of the invention" as used hereinbelow.

The peptides of the invention may be provided in a manner known per se, including of the invention can be prepared in a manner known per se, for instance via peptide synthesis techniques involving the use of protected amino acids, carbodiimide chemistry and/or Fmoc chemistry, which may be carried out using automated equipment. Other techniques include, but are not limited to, solid-phase peptide synthesis, for instance as described by Merrifield, J.Am.Chem.Soc., 85, 2149 (1963). These and other techniques will be known to the skilled person and/or are described in the standard handbooks.

The peptides of the invention can be used in the preparation/formulation of pharmaceutical compositions, and in particular in the preparation/formulation of vaccines.

More in particular, the peptides of the invention can be used in the preparation/formulation of vaccines that are intended to protect a human being against infection by *Bordetella (para)pertussis*. These may be vaccines that are intended to protect solely against pertussis (i.e. so-called "monovalent" or "monocomponent" vaccine), but may also be a combination vaccine (i.e. a so-called "polyvalent" or "polycomponent" vaccine) intended to protect against pertussis and at least one further infectious disease other than pertussis, including but not limited to diphteria, tetanus, polio and/or Hib. Examples of such "combination vaccines" will be clear to the skilled person, and for instance include, but are not limited to, "DTPa" vaccines. ( Also, it will be clear to the skilled person that such (combination) vaccines are often used to immunize/protect infants and children. Thus, as used herein, the term "human being", although not limited to any specific age, will in particular include infants and children.)

The peptides/vaccines of the invention will generally be administered in an amount and/or according to a regimen that will generate/elicite in said human being an immune response, and in particular a protective immune response and/or a detectable immune response as mentioned above. Such a regimen may involve a single administration or two or more administrations, for instance one or more priming immunisations followed by one or more booster immunisations. Suitable regimens will be clear to the skilled person and may essentially be the same as the regimens currently used with known vaccines against pertussis and/or with known combination vaccines.

Usually, the peptide of the invention will be administered in an amount of between 1 and 100 µg, and in particular between 4 and 20 µg/dosis. Thus, a suitable regimen may for instance involve priming immunisations with a dose of 10 µg peptide of the invention, followed by further priming immunisations after 2, 3, 4 and 11 months with the same dose, and a booster administration after 4 years with the same dose.

The peptides/vaccines of the invention may be administered in any suitable manner known per se, for instance orally, transdermally, subcutaneously, intramuscularly, intraperitoneally, intravenously or via the respiratory tract, of which - as will be clear to the skilled person - intramuscular administration is particularly preferred.

The vaccines of the invention may be formulated in any manner known per se - which will usually depend upon the intended route of administration - optionally using one or more pharmacologically acceptable carriers, excipientia or adjuvantia,

Usually, the vaccine of the invention will be in a form intended and/or suitable for injection, such as a solution or suspension of the peptide(s) in water or in a fysiological acceptable buffer or solution. In this respect, the use of the peptides of the invention may also make it possible to provide such vaccines in a form which can be reconstituted - for instance with water or a fysiological acceptable buffer or solution - to provide a preparation suitable for injection, for instance in the form of a dried or freeze-dried powder.

A vaccine of the invention may be suitably packaged in a suitable holder or container, for instance in an ampoule, a bottle or a flask, optionally in combination a leaflet containing product information.

Generally, a vaccine of the invention will contain the peptide(s) of the invention in an amount of between 1 µg and 100 µg, and preferably between 4 µg and 20 µg, usually in the form of a unit dose.

The vaccines of the invention may also comprise one or more immunological adjuvantia acceptable for use in vaccines, such as aluminium phosphate and/or aluminium hydroxide. These may be used in suitable amounts known per se, for instance in amounts known per se from the formulation of known ACV's.

Also, a peptide of the invention may be conjugated with, (covalently) linked to, or otherwise attached to, a functional residue, such as maltose-binding protein, tetanus toxoid or another carrier or immuno-modulating and/or immuno-stimulating group.

In addition, the vaccines of the invention may also contain one or more *Bordetella (para)pertussis* antigens known per se, such as pertussis toxoid, filamenteous hemagglutinin, serotype 2 fimbriae and/or serotype 3 fimbriae, for instance in amounts known per se from acellular vaccines.

Also, in order to provide a combination vaccine as mentioned above, a vaccine of the invention may also contain one or more further pharmaceutically acceptable components that, upon administration to a human being in a suitable amount, can protect said human being against at least one (infectious) disease other than pertussis, for instance diphteria, tetanus, polio and/or Haemophilus influenza b. Usually, such a component will be a suitable antigen known per se, including but not limited to:
- one or more antigens against diphteria such as diphteria toxoid;
- one or more antigens against tetanus such as tetanus toxoid;
- one or more antigens against polio such as inactivated polio virus;
- one or more antigens against *Haemophilus influenza* b.
or a suitable combination thereof. However, it is envisaged that any suitable antigen(s) that may become available after the priority date of the present application may also be used.

Such further antigens will generally be used in amounts that, upon administration to a human being according to a suitable regimen (e.g. as outlined above for the vaccines of the invention), will generate/elicit in said human being an immune response, and in particular a protective immune response and/or a detectable immune response, against the intended disease. Suitable amounts for each specific antigen will be clear to the skilled person, and will usually correspond to the amounts that are normally used in vaccines known per se.

Some preferred but non-limiting combination vaccines of the invention may for instance comprise two or more of the following components in the amounts indicated:

| | |
|---|---|
| peptide of the invention | between 4 µg and 20 µg; |
| pertussis toxoid | between 5 µg and 50 µg; |
| filamentous hemagglutinin | between 5 µg and 50 µg; |
| serotype 2 fimbriae | between 2.5 µg and 50 µg; |
| serotype 3 fimbriae | between 2.5 µg and 50 µg; |
| diphteria toxoid | between 5 µg and 50 µg; |
| tetanus toxoid | between 5 µg and 50 µg; and/or |
| Hib (Haemophilus influenzae type B) | between 2 µg and 10 µg polysaccharide |
| | (RPR: poly-ribosyl ribitiol phosphate). |

Again, such combination vaccines of the invention may be formulated and administered in a manner known per se, e.g. as outlined above.

More generally, the peptide(s) of the invention can be used to replace whole cells *Bordetella pertussis* in known WCVs and/or to replace some or all of the pertussis antigens - including but not limited to (essentially) intact pertactin - currently used in known ACV's,. However, it is also envisaged that the small antigenic peptides of the invention may open up novel types of pertussis vaccines and/or novel ways of formulating and/or administering pertussis vaccines.

As mentioned above, the peptides and vaccines of the invention have the advantage - compared to the currently available pertussis antigens and vaccines - that they can provide - i.e. simultaneously - immunity against (at least) the different strains of *Bordetella pertussis* used in the Experimental Part below, despite the differences between the pertactin proteins associated with the surface of said strains, i.e. due to polymorphisms in the pertactin encoding *prn*-genes between said strains. More broadly, it is expected that the peptides of the invention can be used to provide immunity against all (other) strains of *Bordetella pertussis* currently circulating, and also any strains that may yet be identified and/or that may arise in future; and may also provide protection against (strains of) *Bordetella parapertussis*.

In addition, the use of a small antigenic peptides - compared to the use of the whole cells and/or the antigenic components currently used - may also provide increased stability of the vaccine.
- Some other possible applications of the peptides of the invention include, but are not limited to use in *in vitro* assays and/or in biological assays.

In yet another aspect, the invention relates to (protein) fusion comprising at least one peptide of the invention, linked to at least one further amino acid sequence which is different from the amino acid sequence with which a peptide of the invention is (or may be) natively associated in a naturally occurring pertactin. Such a further protein sequence may optionally be antigenic per se and/or comprise one or more further (antigenic) epitopes, i.e. to provide (protective) immunity against *Bordetella (para)pertussis* and/or another infectious disease, and/or may be an immuno-modulating and/or immuno-stimulating amino acid sequence.

Such fusions may be provided in a manner known per se, such as expression of a nucleotide sequence encoding such a fusion in a suitable host organism - e.g. under the control of a suitable promoter operable in the host organism used - followed by isolation/purification of the fusion from the host organism and/or the culture medium. Suitable techniques for providing nucleotide sequences encoding such fusions, for (heterologous) expression thereof in a host organism, suitable host organisms (such as *Escherichia coli or Bordetella (para)pertussis*) and promoters operable therein (such as T7 RNA polymerase promoter, lac promoter, or promtoters derived from *B. (para)pertussis*, such as the promoter for Omp or filamentous hemagglutinin ), and techniques for isolating the fusion(s) thus expressed will be clear to the skilled person. Preferably, such a fusion is such that, upon administration to a human being in a suitable manner and in a suitable amount, it is capable of generating/eliciting in said human being a protective immune response (in particular against *Bordetella pertussis,* and preferably also against infection by *Bordetella parapertussis*) and/or a detectable immune response, e.g. as outlined above.

The fusions mentioned above may be used in providing/formulating pharmaceutical compositions, and in particular vaccines - i.e. against *Bordetella (para)pertussis -* essentially in the manner outlined above for the peptides of the invention.

In yet another aspect, the invention relates to an antibody directed/generated against a peptide of the invention. As used herein, the term antibody includes *inter alia* polyclonal, monoclonal, chimeric and single chain antibodies, as well as fragments (Fab, Fv, Fa) and an Fab expression library. Furthermore, "humanized" antibodies may be used, for instance as described WO 98/49306. Such antibodies can be obtained in a manner known per se, such as those described in WO 95/32734, WO 96/23882, WO 98/02456, WO 98/41633 and/or WO 98/49306.

For instance, polyclonal antibodies can be obtained by immunizing a suitable host such as a goat, rabbit, sheep, rat, pig, mouse or human with a peptide of the invention, optionally with the use of an immunogenic carrier (such as bovine serum albumin or keyhole limpet hemocyanin) and/or an adjuvant such as Freund's, saponin, ISCOM's, aluminium hydroxide or a similar mineral gel, or keyhole limpet hemocyanin or a similar surface active substance. After an immune response against the peptide of the invention has been raised (usually within 1-7 days), the antibodies can be isolated from blood or serum taken from the immunized animal in a manner known per se, which optionally may involve a step of screening for an antibody with desired properties (i.e. specificity) using known immunoassay techniques, for which reference is again made to for instance WO 96/23882.

Monoclonal antibodies may be produced using continuous cell lines in culture, including hybridoma and similar techniques, again essentially as described in the above cited references. Fab-fragments such as F(ab)₂, Fab' and Fab fragments may be obtained by digestion of an antibody with pepsin or another protease, reducing disulfide-linkages and treatment with papain and a reducing agent, respectively.

The antibodies generated against the peptides of the invention may be used for pharmaceutical and/or diagnostic purposes. For instance, said antibodies may be used to prevent and/or treat infections with *Bordetella (para)pertussis*, e.g. by administering said antibody in a suitable manner known per se - e.g. intravenously, intramuscularly, intranasally - and a suitable amount - e.g. between 25 and 1500 mg/kg body weight - to a human being suffering from, or at risk of, infection by *Bordetella (para)pertussis*.

For this purpose, the antibodies will generally be formulated as a pharmaceutical composition that comprises at least one antibody generated against a peptide of the invention, optionally in combination with at least one pharmaceutically acceptable carrier, excipiens and/or adjuvans; and such compositions form a further aspect of the invention. In particular, such a composition will be in the form of a composition suitable for injection and/or infusion, comprising a solution/suspension of the antibody in water or a suitable fysiological buffer or solution.
Some other uses of antibodies generated against the peptides of the invention may include, but are not limited to and any other pharmaceutical and/or diagnostic applications of antibodies known per se.

In yet another aspect, the invention relates to a polypeptide having the amino acid sequence of a pertactin from which, compared to the sequence of said pertactin:
- the amino acid residues that form the so-called "region 1" have been at least partly, and preferably essentially completely removed; and/or from which,
- the amino acid residues that form the so-called "region 2" have been at least partly, and preferably essentially completely removed.

Such a pertactin from which (at least part of) region 1 and/or from which (at least part of) region 2 has been removed will also be referred to hereinbelow as a *"deleted pertactin*".

In this aspect, the "sequence of a pertactin" may be a naturally occuring (type of) *Bordetella pertussis* pertactin, or may be an analog or variant thereof, including but not limited to synthetic analogs or variants thereof.

In particular, this aspect of the invention relates to such a "deleted pertactin" which has the amino acid sequence of pertactin as shown in SEQ ID NO:6 - or the amino acid sequence of a naturally occurring or synthetic analog or variant thereof - from which:
- at least 5, preferably at least 10, and up to all of the amino acid residues at positions 266 - 290 have been removed; and/or from which
- at least 3, preferably at least 10, and up to all of the amino acid residues at positions 569-603 have been removed.

Even more in particular, this aspect of the invention relates to a such a deleted pertactin having the amino acid sequence of pertactin as shown in SEQ ID NO:6 - or the amino acid sequence of a naturally occurring or synthetic analog or variant thereof - from which one or more amino acid residues are/have been removed (i.e. as outlined above) such that
- from the amino acid residues between positions 260 and 290 of SEQ ID NO:6, at least one repeat "GGAVP" and/or at least one repeat "GGFGP" is/has been removed, and up to all such repeats "GGAVP" and/or "GGFGP" that are present between positions 260 and 290. Preferably, a total of 2, 3, 4 or 5 repeats "GGAVP" and/or "GGFGP" is removed, optionally with one or more further amino acid residues present between positions 260 and 290, e.g. in the form of a contiguous sequence of amino acid residues comprising said repeats; and/or such that:
- from the amino acid residues between positions 569 and 603 of SEQ ID NO:6, at least one motif "PQP" is/has been removed, and up to all motifs "PQP" that are present between positions 569 and 603.

Preferably, 1, 2, 3, 4 or 5 motifs PQP are removed; optionally with one or more further amino acid residues present between positions 569 and 603, e.g. in the form of a contiguous sequence of amino acid residues comprising said PQP motifs.

Most preferably, the deleted pertactin has the amino acid sequence of pertactin as shown in SEQ ID NO:6 - or the amino acid sequence of a naturally occurring or synthetic analog and/or variant thereof - from which the following amino acid residues have been removed:
- the residues from Gly at position 266 up to and including Pro at position 290; or
- the residues from Gly at position 270 up to and including Pro at position 285; and/or from which any of the following amino acid residues have been removed.
- the residues from Ala at position 569 up to and including Ala at position 603; or
- the residues from Pro at position 572 up to and including Gln at position 599; or
- the residues from Pro at position 575 up to and including Pro at position 596; or
- the residues from Ala at position 578 up to and including Glu at position 594; or
- the residues from Pro at position 581 up to and including Pro at position 591; or
- the residues from Gln at position 584 up to and including Pro at position 588.

In addition to the one or more amino acid residues from region 1 and/or from region 2 mentioned above, also one more more of the amino acid residues flanking region 1 and/or region 2 may (also) be removed, e.g. from Thr at position 206 up to and including His at position 340; and/or from Asn at position 569 up to and including Gly at position 653.

As mentioned above, the deleted pertactin may have been derived from the sequence of SEQ ID NO:6, or from a naturally occurring and/or synthetic analog or variant thereof, i.e. an analog or variant in, from or to which one or more amino acid residues have been inserted, deleted, substituted (preferably as a "conservative" substitution) or added.

Preferably, such an analog or variant has a percentage "(amino acid) sequence homology" of more than 30%, preferably 40%, more preferably 50 %, even more preferably 60%, still more preferably 70%, yet more preferably more than 80%, and most preferably more than 90%; in which:
- the percentage of sequence homology is calculated by [*number of amino acid residues in the amino acid sequence of the analog or variant which are the same as the amino acid residue at the corresponding position of the sequence of SEQ ID NO:6*] divided by [*the total number of amino acid residues of SEQ ID NO:6*] and multiplied by 100%; and in which:
- the amino acid residues or positions which are removed from the sequence of SEQ ID NO:6 to provide the deleted pertactin (i.e. as outlined above) are not taken into account;
- each deletion, insertion, substitution, addition of a single amino acid residue is taken as a difference at a single amino acid position.

Alternatively, the percentage of "sequence homology" may also be determined using suitable computer algorithms such as BLAST or PC-Gene at standard settings.

Thus, a "deleted pertactin" as described above may also comprise or be derived from one or more parts or fragments of a pertactin, but preferably only as long as the resulting deleted pertactin still meets the requirements on sequence homology outlined above.
Preferably, such a deleted pertactin comprises at least 200 amino acids, preferably at least 400 amino acids, more preferably at least 600 amino acids, and even more preferably at least 800 amino acids.

Also, to provide a deleted pertactin according to this aspect of the invention, it may be possible to replace one or more of the amino acids that are deleted to provide the deleted pertactin - i.e. as outlined above - with one or more other amino acid residues, most preferably however not such that the amino acid residues that replace the deleted amino acid residues provide essentially the same biological - e.g. immunological - effect (optionally in conjunction with the remaining amino acid residues from the pertactin sequence) as the deleted amino acid residues. Again, preferably, such a polypeptide should meet the requirements as to sequence homology outlined above, in which the amino acid residues that are substituted for the amino acid residues that are deleted to provide the deleted pertactin are not taken into account.

Preferably, any "deleted pertactin" as outlined above is such that, upon administration to a human being in a suitable manner and in a suitable amount, it is capable of generating/eliciting in said human being an immune response.

More preferably, any such deleted pertactin is such that, upon administration to a human being in a suitable manner and in a suitable amount, it is capable of generating/eliciting in said human being a protective immune response, and in particular an immune response which protects said human being against infection by *Bordetella pertussis,* and preferably also against infection by *Bordetella parapertussis*, as may be determined using one or more of the techniques referred to above.

Also, any such deleted pertactin is preferably such that, upon administration to a human being in a suitable manner and in a suitable amount, it is capable of generating/eliciting in said human being a "detectable immune response", which for an analog may also include the generation against antibodies against the specific analog used, and as again may be determined using one or more of the techniques referred to above.

A deleted pertactin as outlined above may be provided in a manner known per se, such as expression of a nucleotide sequence encoding such a deleted pertactin in a suitable host organism - e.g. under the control of a suitable promoter operable in the host organism used - followed by isolation/purification of the fusion from the host organism and/or the culture medium. Again, suitable techniques for providing nucleotide sequences encoding such deleted pertactins, for (heterologous) expression thereof in a host organism, suitable host organisms (such as *Escherichia coli or Bordetella (para)pertussis*) and promoters operable therein (such as T7 RNA polymerase promoter, lac promoter, or promtoters derived from *B.(para)pertussis*, such as the promoter for Omp or filamentous hemagglutinin), and techniques for isolating the deleted pertactin(s) will be clear to the skilled person.

The deleted pertactins mentioned above may again be used as antigenic components for use in providing/formulating pharmaceutical compositions, and in particular in providing/formulating vaccines - i.e. against *Bordetella (para)pertussis -* essentially in the manner outlined above for the peptides of the invention.

In such applications, the deleted pertactins may provide the same advantages as described above for (the vaccines containing) the peptides of the invention. In particular, as with the peptides of the invention, the deleted pertactins of the invention may circumvent the mechanisms evolved by the bacteria to direct the immune response towards conformational, variable, epitopes, and thus provide cross-immunity, also against (strains of) *Bordetella (para)pertussis*.

The invention also relates to antibodies generated against a deleted pertactin as outlined above, and to pharmaceutical compositions containing such antibodies. Such antibodies and such compositions may be obtained and/or used essentially as outlined above for the antibodies against the peptides of the invention.

In yet another aspect, the invention relates to a pharmaceutical composition, and in particular to a vaccine - i.e. against *Bordetella (para)pertussis -* that comprises at least one peptide of the invention in combination with at least one deleted pertactin as described above. Such pharmaceutical compositions/vaccines may also comprise one or more of further constituents - e.g. those outlined hereinabove for the vaccines of the invention - to provide a monovalent and/or polyvalent vaccine.

Although the invention has been described hereinabove mainly with respect to human applications, it should be noted that, in yet another aspect, the peptides of the invention, the deleted pertactins of the invention and/or the antibodies described above may also be used (to provide a composition suitable and/or intended) for veterinary purposes, and in particular to provide veterinary vaccines against infections by *Bordetella parapertussis* and *Bordetella bronchiseptica* in mammals such as cats, dogs, pigs, cattle. The preparation and use of such vaccines, which again may be monovalent vaccines or polyvalent, will be clear to the skilled person, also taking into account the further disclosure herein.

The invention will now be illustrated by means of the following non-limiting Experimental Part, and by means of the non-limiting Figures, in which:
- Figure 1 is a schematic drawing showing the structure of the pertactin gene, and the location of polymorphic sites. The proteolytic cleavage sites are indicated with asterisks and the parts of the gene resulting in the products P.69 and P.30 are indicated. The RGD sequence involved in attachment to host receptors has been shaded. Note the presence of 3 types of repeats GGavP, GGfgP and GGgvP in region 1. Dashes indicate gaps in the sequence introduced to increase the number of matches. Numbers indicate the position of amino acids in Prn1 relative to the N-terminus of the unprocessed molecule.

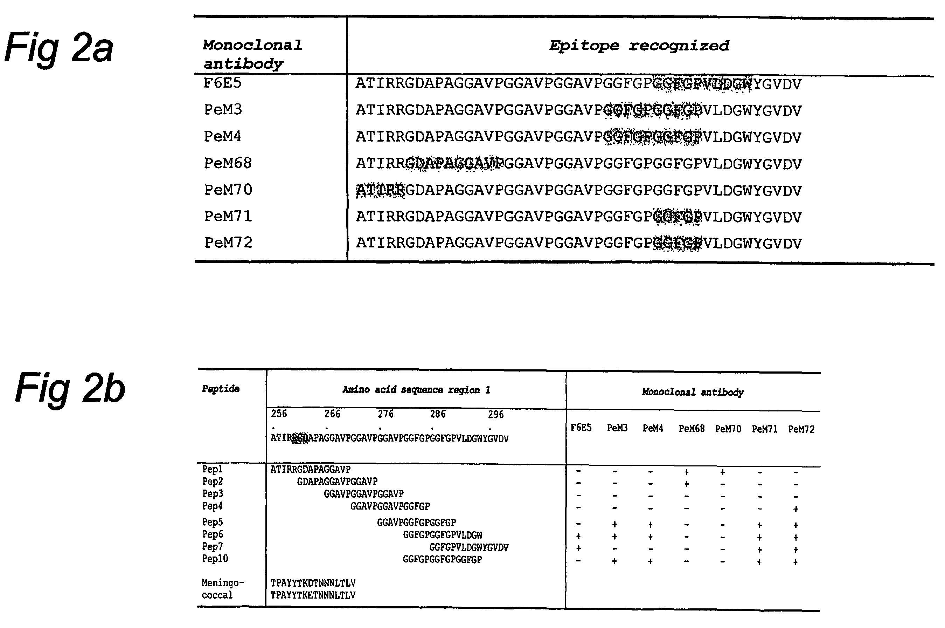

- Figures 2A and 2B are tables showing the amino acid sequence of peptides used for epitope-mapping and vaccination. (A) Epitope mapping of monoclonal antibodies specific for region 1 of pertactin. The sequence of each individual peptide is given, the RGD motive, involved in adherence, has been shaded. Binding of the monoclonal antibodies to the peptides is indicated by "-" (no binding) and "+" (binding). Peptides used for vaccination were conjugated to tetanus toxoid. The meningococcal peptides were used as negative controls in the vaccination experiments. (B) Epitopes bound by monoclonal antibodies. Shading in the region 1 sequence indicates epitopes bound by each individual monoclonal antibody.

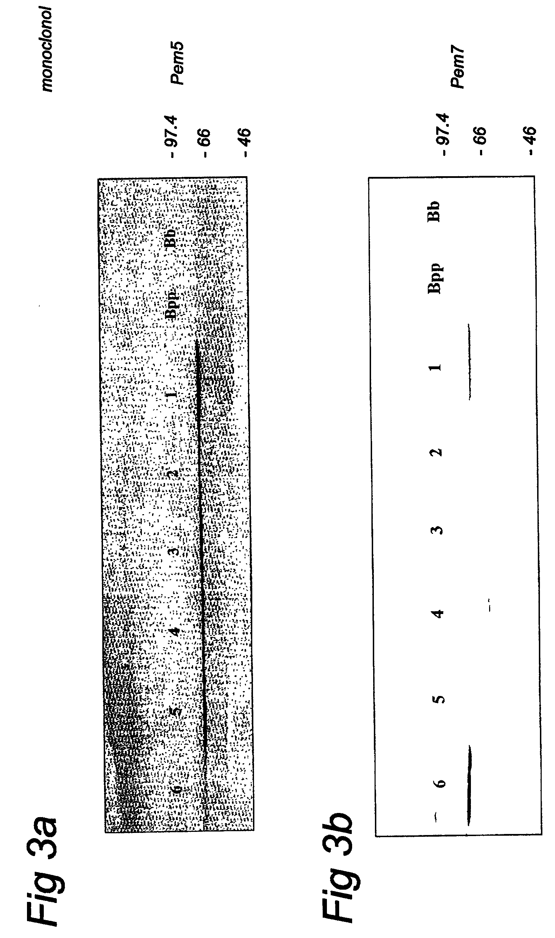

- Figures 3A and 3B represent immunoblotting gels showing differential binding of monoclonal antibodies to distinct pertactin types. Cell lysates from B. pertussis strains (expressing Prn1-6), B. bronchiseptica (Bb) and B. parapertussis (Bpp) were analyzed by immunoblotting, using anti-pertactin monoclonal antibodies PeM5, (panel A) or PeM7, (panel B). The numbers above the lanes refer to the pertactin type. Position of molecular weight markers are indicated.

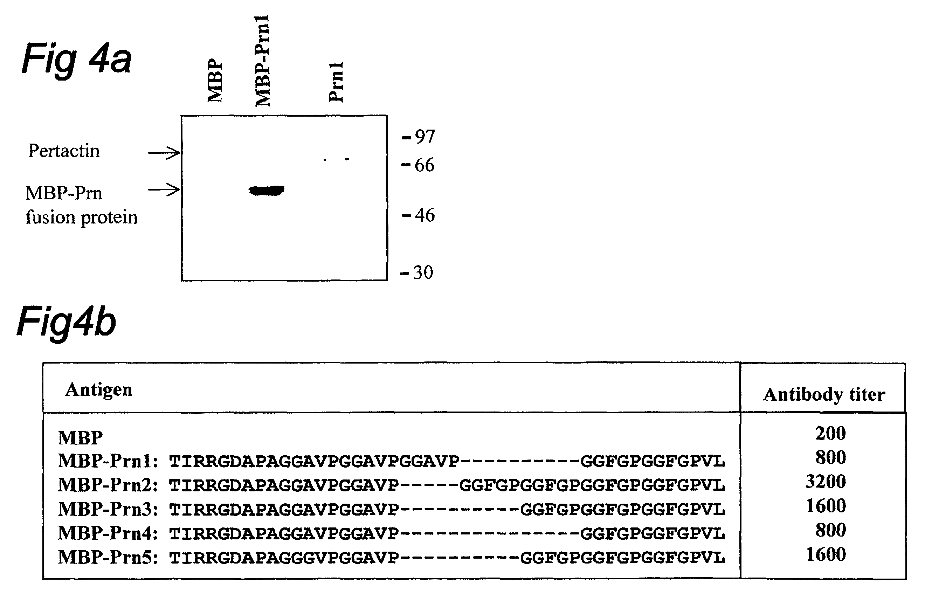

- Figure 4A represents and immunoblotting gel, and Figure 4b is a graph, showing that region 1 of pertactin is immunogenic in mice. Mice were immunized with Prn1 and the ability of the antiserum to bind to MBP fusion proteins with region 1 derived from Prn1 to Prn6 was assessed. (A) Immunoblot with anti-Prn1 mouse serum. Only the result obtained with MBP-Prn1 is shown. Identical results were obtained with MBP-Prn2 to MBP-Prn5. Equal amounts of MBP and MBP-Prn1 were loaded on the gel. Positions of pertactin and MBP-Prn1 are indicated. (B) ELISA with anti-Prn1 mouse serum. Antibody titers in serum to MBP fusion proteins, and the sequence of the pertactin region fused to MBP, are shown.

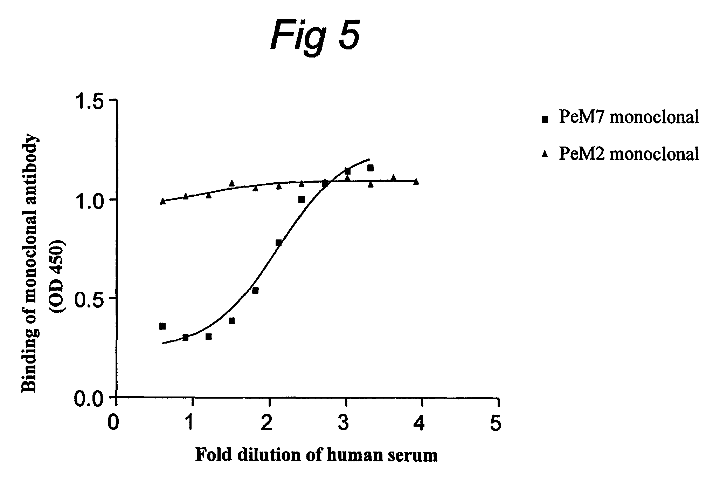

- Figure 5 is a graph showing that serum from pertussis patients harbors antibodies directed against region 1. ELISA plates were coated with Prn1. Binding of monoclonal antibodies PeM7 (directed against region 1) or PeM2 (directed against an epitope present in all pertactin variants) was allowed after pre-incubation with two-fold dilutions of human serum. Human serum was from one pertussis patient, similar results were obtained with a pool of sera from different pertussis patients.

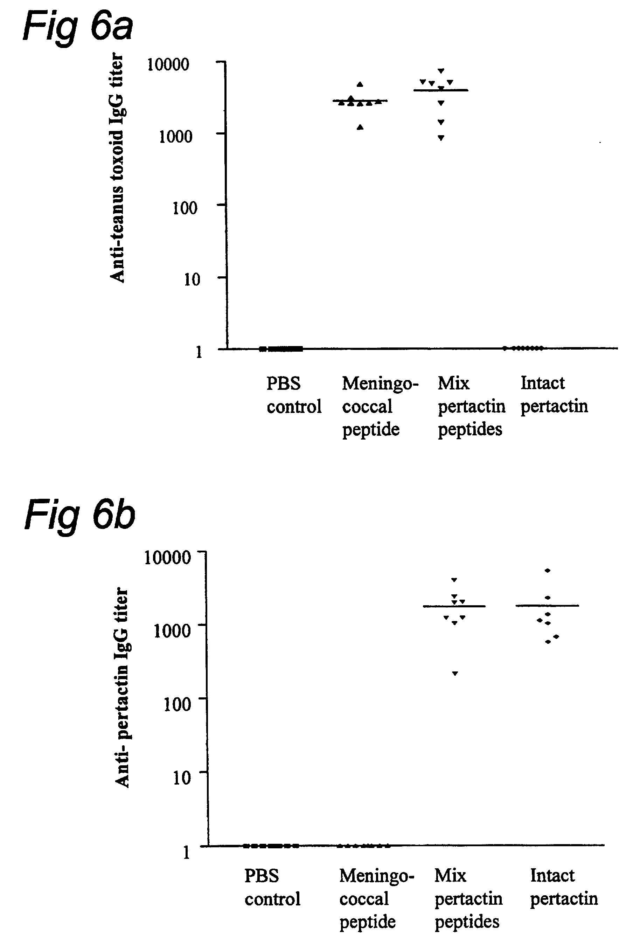

- Figures 6A-6D are graphs showing that immunization with peptides derived from pertactin region 1 protects against intranasal B. pertussis infection. Mice were immunized with PBS, a meningococcal peptide, a mixture of peptides derived from region 1, or with pertactin (Prn1). Peptides were conjugated to tetanus toxoid. After immunization, mice were intranasally infected with B. pertussis strain B213 expressing Prn1. The amount of bacteria in lungs and trachea, and antibody titers, were determined 3 days post-infection. (A) Anti-tetanus toxoid IgG titers. (B) Anti-Prn1 IgG titers. (C) CFU in the lungs. (D) CFU in trachea. The thin line indicates the mean. P values are indicated. The experiment was performed three times and a representative result is shown.

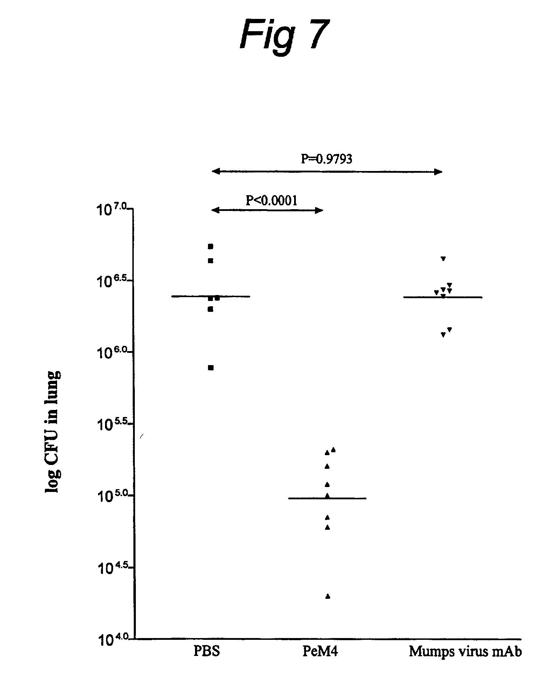

- Figure 7 is a graph showing the passive protection against B. pertussis infection by a monoclonal antibody directed against region 1 of pertactin. Mice were immunized passively by administration PeM4, a monoclonal antibody directed against mumps virus, or PBS, respectively. After immunization, mice were intranasally infected with B. pertussis strain B213 (Prn1). The amount of bacteria in lungs and trachea, was determined 3 days post-infection. The thin line indicates the mean. P values are indicated. The experiment was performed three times and a representative result is shown.

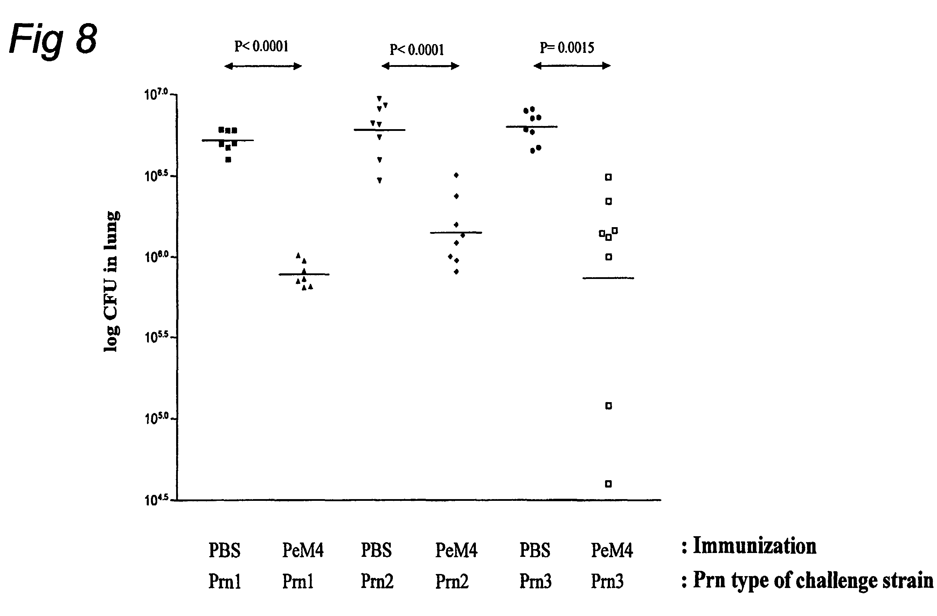

- Figure 8 is a graph showing that a monoclonal antibody (PeM4) against a conserved epitope in region 1 confers cross-immunity against B. pertussis strains with distinct pertactin variants. The experiment was performed twice, and a representative result is shown. See the legend to Fig. 7 for experimental details;

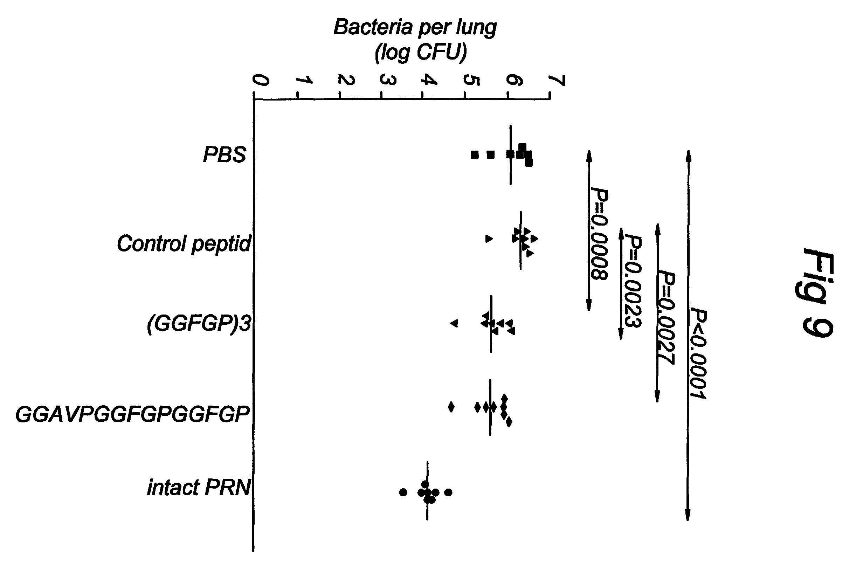

- Figure 9 is a graph showing protection against intranasal *B. pertussis* infection by immunization with peptides derived from pertactin region 1. Mice were immunized with PBS, a meningococcal peptide (control), peptide GGFGP-GGFGP-GGFGP-, peptide GGAVP-GGFGP-GGFGP, or with pertactin (Prn1). Peptides were conjugated to tetanus toxoid. After immunization, mice were intranasally infected with B. pertussis strain B213 expressing Prn1. The amount of bacteria in lungs and trachea was determined 3 days post-infection.

### EXPERIMENTAL PART

### EXAMPLE 1

### 1. Introduction

*Bordetella pertussis* is the principal etiological agent of whooping cough or pertussis, a respiratory disease that is most severe in infants and young children. The second causative agent of pertussis, *Bordetella parapertussis*, is isolated less frequently ( 1-5 %) from patients. In the pre-vaccination era, nearly every child contracted whooping cough and this infection was a major cause of infant death throughout the world. Since about forty years, wide spread immunizations of young children with a whole cell pertussis vaccine (WCV) have been successful in controlling the disease. However, there is evidence that the incidence of pertussis has increased in recent years in a number of countries such as Australia, Canada, USA, and The Netherlands, despite a high vaccination coverage.

Recently it was reported that two *B. pertussis* antigens implicated in protective immunity, pertussis toxin and pertactin are polymorphic (Mooi et al (1998), *supra*). Three protein variants of pertactin and pertussis toxin were found in Dutch clinical isolates collected from 1949-1996. Temporal trends in the frequencies of the pertactin and pertussis toxin variants indicated a divergence between vaccine strains and clinical isolates (Mooi et al (1998); and Mooi et al (1999), both *supra*). These studies suggested that expansion of strains that are antigenically distinct from vaccine strains might be one of the causes for the re-emergence of pertussis.

A number of studies have shown that the B. pertussis proteins fimbriae, filamentous hemagglutinin, pertussis toxin, and pertactin can induce protection in both animals and humans. Therefore, these proteins have been included in acellular pertussis vaccines. The importance of pertactin as a protective antigen is particularly convincing: the presence of antibodies against pertactin correlate with clinical protection (Cherry et al., Vaccine (1998) 16:1901-1906; and Storsaeter et al., Vaccine (1998) 16:1907-1916). Furthermore acellular vaccines containing pertactin have been reported to give better protection than vaccines without this component (Hewlett, Pediatr. Infect. Dis. J. (1997) 16:S78-84; Plotkin and Cadoz, Acellular vaccine efficacy trials (1997) 16:913-914). An important role of pertactin in protective immunity is also suggested by the fact that it is one of the few polymorphic surface molecules found in B. pertussis to date (Mooi et al. (1998), *supra*). Pertactin belongs to a family of secreted proteins designated autotransporter proteins. It is produced as a large (910-amino-acid) precursor molecule, which is proteolytically processed at its N- and C-terminus to produce P.69 and P.30 (Fig. 1), which are located at the cell surface and in the outer membrane, respectively. P.69/pertactin (henceforth designated as pertactin) is included in acellular vaccines. It contains the amino acid triplet arginine-glycine-aspartic acid (RGD), a sequence motif which functions as a cell-binding site in a number of mammalian proteins, and it has been shown that the pertactin RGD sequence is also involved in adherence to host cells.

Polymorphism in pertactin is mainly found in 2 regions comprised of proline rich repeated sequences, designated region 1 and region 2, respectively (Fig. 1). Most variation is found in region 1 which is located proximate to the N-terminus, flanks the RGD sequence, and consists of repeats of the glycine-glycine-x-x-proline (GGxxP) motif. The crystal structure of pertactin predicts that both the RGD sequence and the GGxxP motif are exposed at the surface of pertactin. Region 2 is located more towards the C-terminus and, is comprised of repeats of proline-glutamine-proline (PQP).

Although epidemiological studies suggest that variation in surface antigens increases the fitness of *B. pertussis* in immunized populations and can affect vaccine efficacy, no direct evidence has yet been presented for its immunological relevance. Here, the immunological importance of the polymorphism in pertactin was investigated. Since variation in pertactin was essentially restricted to region 1, this domain was focused upon. It is shown that region 1 of pertactin is indeed antigenic. Vaccination with peptides derived from region 1 protected mice against a challenge with *B. pertussis.* Furthermore a monoclonal antibody directed against region 1 was protective. Significantly, this monoclonal antibody recognized a conserved epitope and was able to protect against strains with distinct pertactin variants.

### 2. Methods

### 2A. Bacterial strains and plasmids:

*B. pertussis, Bordetella parapertussis* and *Bordetella bronchiseptica* strains used in this study are indicated in Table I. *Bordetella* strains were grown on Bordet-Gengou (BG) agar (Difco Catalog no.0048-17-5, Detroit, USA) supplemented with 1% glycerol and 15 % sheep blood at 35 °C for 3 days. *Escherichia coli* strains DH5α and BL21 (DE3) were used for the propagation of plasmids. *E. coli* strains were routinely grown in L-broth or on L-agar supplemented with antibiotics. pMAL-c2 vector was purchased from New England Biolabs, Beverly, MA, USA.

### 2B. DNA sequencing:

The pertactin gene of all strains used in this study were sequenced previously or during this study (vide Li et al.; Mastrantonio et al.; Mooi et al.(1998) and Mooi et al. (1999), all *supra*; vide also Table I). DNA was isolated using standard procedures. DNA sequencing was performed by direct sequencing of PCR products. Sequencing was carried out using the ABI prism dye terminator cycle sequencing ready kit (Perkin Elmer-Applied Biosystems, Foster City, Calif., USA) and the products were analyzed on a 373 ABI DNA sequencer (Perkin Elmer).

### 2C. Construction of maltose binding protein containing the pertactin polymorphic region 1:

Part of the open reading frame of the *prn1* gene, coding for the amino acid sequence of SEQ ID NO:1 (defined as region 1) TIRRGDAPAGGAVPGGAVPGGAVPGGFGPGGFGPVL (SEQ ID NO:1) was expressed in *E. coli* as a fusion with the maltose binding protein (MBP) by cloning the corresponding pertactin DNA sequence in the pMAL-c2 vector (New England Biolabs, Beverly, USA). The pertactin sequence was obtained by PCR with the following two primers: 
5'-CGGGATCCACGATACGGCGCGGGGAC-3'    (SEQ ID NO:7);
5'-GCTCTAGAGAGGACGGGACCGAAGCC-3'    (SEQ ID NO:8)
 and with chromosomal DNA of *B. pertussis* strain B5. In the primers the *Bam*HI and *Xba*I restriction sites, introduced to facilitate cloning, are underlined, whereas pertactin-derived sequences are indicated in bold. PCR products derived from *prn2, prn3, prn4*, and *prn5* were obtained in a similar way using the appropriate strains; B394, B365, B705 or B1148, respectively (Table I). Gel-purified PCR products were digested with *Bam*HI and *Xba*I and subsequently ligated in *Bam*HI-*Xba*I digested pMAL-c2. All plasmid constructs were checked by DNA sequencing.

### 2D. Purification of maltose binding protein-pertactin fusion proteins:

500 ml *E. coli* cultures (BL21) carrying pMAL-c2 derivatives were grown in L-broth medium containing ampicillin (200 µg/ml) and 1% glucose at 37 °C. At an OD₆₀₀=0.5 expression of the MBP-fusion protein was induced by the addition of isopropyl-β-D-thiogalactopyranoside (IPTG) to a final concentration of 0.3 mM. After induction, growth was continued for 2 hrs and cells were harvested by centrifugation at 13000 x g for 20 minutes. The pellet was resuspended in lysis buffer (= 10 mM NaPO₄, pH 7.2, 0.5 M NaCl, 0.25% Tween 20, 10 mM EDTA and 0.1 mM PMSF), and stored at -20 °C overnight. After thawing, the cell suspension was sonicated in ice for 4 x 30 seconds with 2 minutes interval (Branson Sonifier 250, normal tip, 50 % output). Extracts were diluted to 100 ml with column buffer A (20 mM Tris, 200 mM NaCl, 1 mM EDTA) and applied on a 5 ml amylose column. After extensive washing with buffer A, bound proteins were eluted with buffer A, supplemented with 10 mM Maltose, and fractions of 1 ml were collected. Purity was >99%, as estimated by SDS-PAGE followed by Coomassie Brilliant Blue (Serva) staining.

### 2E. Production of polyclonal and monoclonal antibodies:

Polyclonal antiserum against pertactin was prepared by repeated injection (on day 0 and day 28) of BALB/c mice with 5 µg Prn1 kindly provided by Chiron-Biocine SpA (Siena, Italy) in 0.35% Alhydrogel in PBS. Mice were bled on day 42. Monoclonal antibodies PeM1, PeM2, PeM3, PeM4, PeM5, PeM6 and PeM7 were generated by injection of BALB/c mice subcutaneously three times with purified pertactin mixed with Specol. PeM70, PeM71 and PeM72 were generated similarly but instead of Prn1, MBP-Prn1 was used as the antigen. For the production of PeM68, mice were injected with MBP-Prn5. PeM80, PeM84 and PeM85 were generated using Prn5. Three days before the fusion, mice were boosted intravenously. Spleens cells were fused with mouse myeloma cells SP2/0 using 50 % PEG 1500 (Boehringer, Mannheim, Germany). Hybridomas secreting antibody to pertactin were selected by ELISA and cloned twice by limiting dilution. Monoclonal antibodies were purified by protein-G affinity chromatography (Pharmacia, Uppsala, Sweden).

### 2F. Synthesis of peptides:

Peptides were assembled by using an automated multiple peptide synthesizer, equipped with a 48-column reaction block (AMS 422, ABIMED Analysen-Technik Gmbh, Langenfeld, Germany), as described by Brugghe et al., (1994), Int. J. Peptide Protein Res. 43:166-172. Peptides used for epitope mapping were N-terminally acetylated multiple (i.e. octameric) antigenic peptides (MAPs), prepared as described previously (Rouppe van der Voort et al., FEMS Immunol. Med. Microbiol. (1997) 17:139-148). The amino acid sequences of the pertactin region 1 specific peptides are listed in Fig. 2a. Peptides used for immunization contained the same amino acid sequence but were monomeric. These peptides were N-terminally elongated with an S-acetylmercaptoacetyl group (Drijfhout et al., Anal. Biochem. (1990) 187:349-354) and conjugated to tetanus toxoid (Brugghe et al., (1994), Int. J. Peptide Protein Res. 43:166-172; and VanderLey et al., Infect. Immun. (1991) 59:2963-2971). Control peptide-tetanus toxoid conjugates used for immunizations were derived from meningococcal PorA.

### 2G. Immunoblotting:

Cell-free extracts of B. *pertussis* (strains B391, B596, B647, B705, B935, B1120, B14 and B24), or purified MBP-fusion proteins, were analyzed by sodium dodecyl sulfate-polyacrylamide (10 %) gel electrophoresis (SDS-PAGE) as described by Laemmli, Nature (Lond.) (1970) 227:680. Proteins were transferred to nitrocellulose filters by electroblotting (Biometra semid-dry blotting) using 5mA/cm² for 30 minutes. After blocking with 0.5 % protifar non-fat dry milk, 0.1 % BSA and 0.1 % Tween 20 in PBS, the filters were incubated for 2 hrs with antiserum or monoclonal antibodies. Filters were washed 3 times for 10 minutes in PBS containing 1% Tween (PBS-1 % Tween 20) and subsequently incubated for 1 hr with goat anti-rabbit total IgG conjugated to horse-radish peroxidase (HRP) in PBS. After two 10 minutes washes in PBS supplemented with 1 % Tween 20, followed by a 10 min wash in PBS, filters were incubated with the ECL substrate (AmershamPharmacia Biotech) and bound HRP was visualized by exposing a sheet of autoradiography film (Hyperfilm-ECL).

### 2H. Epitope mapping:

Polystyrene 96-well plates (Greiner ELISA) were coated for 2 h at 37 °C with 100µl/well multimeric peptides (2 µg/ml) dissolved in PBS. Plates were washed 4 times with 200 µl PBS supplemented with 0.05 % Tween 20 (PBST) per well using the TitertekPlus M96V washer and blocked by incubation with PBST supplemented with 0.5 % Bovine serum albumin (BSA, Sigma) for 1 hr at room temperature. Monoclonal antibodies (murine), diluted in 0.5 % BSA in PBST were added to the wells and incubated for 2 h at 37 °C followed by four washings as described above. Bound antibodies were detected using HRP-conjugated anti-mouse total IgG (Cappel, Organon Technica). Mouse IgG subclasses were determined by using mouse monoclonal antibody isotyping kit (Isostrip, Boehringer). Extinctions (OD₄₅₀) were measured with a BioTek plate reader (EL312e, BioTek Instr.).

### 2I. Immunization and challenge of mice:

B. pertussis strains B213 (Tohama) (prn1), B1296, (prn2) or B1400 (prn3) were grown on BG agar supplemented with streptomycin (30 µg/ml) at 35 °C for 3 days. Subsequently the bacteria were plated on BG agar plates without streptomycin. After 3 days, bacteria were harvested and resuspended in Verwey medium (Verwey et al., J. Bacteriol. (1949) 50:127-134) to a concentration of 5x10⁸ bacteria/ml. An aliquot of the final suspension was diluted and plated to determine the CFU of the challenge inoculum. Groups of 8 female BALB/c mice (RIVM or Harlan) were used for immunization. For passive immunization, 250 µg of a purified monoclonal antibody was injected intravenously into 4 wks old mice. Mice were infected 24 h later. For active immunization, 3 wk old mice were immunized subcutaneously, on day 0, day 14 and day 28 with 0.5 ml PBS containing 20 µg Quil A (Spikoside, Iscotec AB, Lulea, Sweden) and 50 µg tetanus-conjugated peptide. When a mix of 7 peptides was used, equal amounts of each peptide (i.e. 6.25 µg) were present in the vaccine except for peptide 4 of which 12.50 µg was present. Mice were infected 14 days after the last immunization. For infection mice were lightly anesthetized with ether, and a drop of 20 µl of the inoculum was placed on top of each nostril and allowed to be inhaled by the animal. Mice were infected with a total amount of 2x10⁷ B. pertussis cells. Three days after infection mice were sacrificed by intraperitoneal injection of an overdose of barbiturate (Nembutal, Sanofi/Algin) and lungs, trachea and blood were collected. For the determination of bacterial colonization, lungs were homogenized in 900 µl Verwey medium for 10 seconds at 20.000 rpm using a handheld homogenizer (pro scientific, pro200). The trachea was excised and vortexed in 200 µl Verwey medium with 5 glass pearls (diameter 3 mm) for 30 seconds. Appropriate dilutions of the homogenates were plated on BG agar plates supplemented with streptomycin (30 µg/ml). The number of CFU was counted after 4 days of incubation at 35 °C. Levels of serum antibody directed against antigens used for vaccination was determined by ELISA as described above. Plates were coated using tetanus toxoid (2 µg/ml) or Prn1 (2 µg/ml). Extinctions were measured with a BioTek plate reader (EL312e, BioTek Instr.) and titers were calculated with Kineticalc (KC4, BioLyse).

### 2J. Blocking ELISA:

Blocking Elisa was performed essentially as described in Berbers et al., (1993) J. of Virological Methods 42:155-168. Plates were coated with Prn1 (2 µg/ml) as described above. After washing and blocking, the wells were incubated with 100 µl of serial two-fold dilutions of human serum diluted in 0.5 % BSA in PBST for 2 h at 22 °C. Human sera were from two sources. One serum was from a vaccinated child that was recently infected with pertussis. The serum-sample was taken 47 days after the onset of the disease. The second serum sample comprises a mix of sera from several recently infected pertussis patients which were fully vaccinated. After washing the plates, a pertactin-specific murine monoclonal antibodies PeM7, PeM2, PeM5 or PeM6 was added and the plates were incubated for 2 h at 22°C. After the plates were washed thoroughly, they were developed and read as described above.

### 3. Results

### 3A. Characterization of monoclonal antibodies against pertactin:

Previously, the art identified 6 different pertactin types in *B. pertussis* strains circulating in Europe (vide Mastrantonio et al.; Mooi et al. (1998) and Mooi et al. (199), all *supra*). Variation between these pertactin types is mainly restricted to region 1, and consists of deletions or insertions of the repeat unit GGxxP (Fig. 1). Herein, it was investigated whether the polymorphism in region 1 affected antibody binding, i.e. represented antigenic variation. To this purpose several monoclonal antibodies, were raised using pertactin variants and MBP-fusion proteins with region 1 derived from different pertactin variants.

Binding of the monoclonal antibodies to the 6 *B. pertussis* pertactin types and the homologous proteins derived from the closely related species *B. bronchiseptica* and *B. parapertussis* was analyzed by immunoblotting. The amount of pertactin loaded on the gels was standardized using monoclonal antibody E4D7 which is directed against a conserved part of pertactin (i.e. APQPPAGR, which is located close to region 2) by the general method described by Tam 1998, *supra.* The pertactin genes of all strains used were sequenced completely to identify polymorphism outside region 1. Compared to *prn1*, the *B. bronchiseptica* and *B. parapertussis* pertactin genes revealed amino acid substitutions over the whole length of the gene (not shown). However, within *B. pertussis* only *prn6* showed polymorphism outside region 1; compared to *prn1, prn6* contained four amino acid substitutions at amino acid positions 102, 337, 532, 853 and a deletion of 3 amino acids in region 2 (Fig. 1). Of the 14 monoclonal antibodies tested, 7 cross-reacted with pertactin derived from *B. bronchisceptica* and *B. parapertussis* (Table 2).

Out of the 14 monoclonal antibodies, 2 (Pem5 and PeM7) showed differential binding to *B.pertussis* pertactin (Fig. 3, Table 2). PeM5 showed highest and lowest binding to Prn1 and Prn6, respectively. Intermediate binding was observed with Prn2-5. This indicates that PeM5 recognizes an epitope that comprises part of region 1. In region 1, Prn1 and Prn6 differ in 1 amino acid, at position 268 (Fig. 1), implicating this residue as part of the structure of the PeM5 epitope.

PeM7, although raised with Prn1, showed a much stronger reaction with Prn6 compared to Prn1. Weak binding was observed with Prn2-5 (Fig. 3). Since, with the exception of Prn6, the different pertactin proteins do not vary outside region 1, the differential binding of monoclonal antibodies is due to recognition of an epitope encompassing region 1. PeM5 and Pem7 did not bind to pertactin from *B.bronchiseptica* and *B.parapertussis* (Fig. 3). Taken together, these results indicate that variation in region 1 affects antibody binding.

The ability of the monoclonal antibodies to bind to region 1 was also studied with MBP-fusion proteins harboring region 1 derived from the pertactin variants Prn1-5. Binding was assessed by immunoblotting. Of the 14 monoclonal antibodies tested, 7 (PeM3, PeM4, PeM68, PeM70, PeM71, PeM72 and F6E5) bound to the fusion proteins but not to MBP (Table 2). No quantitative differences were observed between these 7 monoclonal antibodies with respect to binding to the MBP-fusion proteins. The monoclonal antibodies that did not bind to the MBP fusion proteins may recognize an epitope located outside region 1. It is also possible that these monoclonal antibodies recognize a conformational epitope encompassing region 1, which folds differently in a MBP fusion protein compared to pertactin. In fact this is probably the case with PeM5 and PeM7, which do not bind to the MBP fusion proteins, although the type specificity of these monoclonal antibodies indicated that they bound to region 1 of pertactin. Significantly, these data indicate that a large number (8) of all (14) monoclonal antibody raised against intact pertactin were directed against region 1, suggesting it represents an immunodominant region in mice.

### 3B. Epitope mapping:

To delineate the epitopes recognized by the monoclonal antibodies described above, a set of 8 overlapping peptides corresponding to the region 1 (Pep1 to 7 and Pep10) was used (Fig. 2a). The 7 monoclonal antibodies (F6E5, PeM3, PeM4, PeM68, PeM70, PeM71 and PeM72) which bound to the fusion proteins also bound to the peptides (Fig. 2a). The monoclonal antibody F6E5 was previously reported to bind a pertactin peptide of 128 amino acids encompassing the RGD and the GGxxP repeat (Charles et al., Eur. J. Immunol. (1991), 21:1147-1153). Herein, it is shown that F6E5 bound to peptides 6 and 7 only, defining the sequence GGFGPVLDGW as its epitope. Based on their differential binding to the peptides the epitopes of the other monoclonal antibodies were also delineated, and are shown in Fig. 2b. The epitopes comprised of GGFGPGGFGP and GGFGP were each recognized by two monoclonal antibodies, respectively, PeM3, PeM4 and PeM71, PeM72. Epitopes for PeM68 and PeM70 were identified as GDAPAGGAVP and ATIRR, respectively. PeM5 and PeM7 did not bind to the peptides, which is consistent with the assumption that they recognize a conformational epitope.

### 3C. Antibodies against region 1 are induced in mice and humans:

In order to further confirm that antibodies are induced against region 1 of pertactin, mice were immunized with Prn1, and the resulting antiserum was tested with immunoblotting and ELISA (Fig. 4). As antigen MBP-fusion proteins harboring region 1 from pertactin variants Prn1-5 were used. Immunoblotting detected mouse antibodies against all MBP-fusion proteins, but not MBP, indicating the presence of antibodies against region 1 (Fig. 4a). Antibody titers against the different pertactin region 1 sequences were assessed by ELISA (Fig. 4b). The results showed that, although this serum was raised against the Prn1 protein, the highest binding was observed to MBP-Prn2, whereas the lowest binding was found to MBP-Prn1 and MBP-Prn4. An intermediate level of binding was observed with MBP-Prn3 and MBP-Prn5. The level of binding correlated best with the number of GGFGP repeats (Fig. 4b), suggesting that after immunization with Prn1 a substantial fraction of the antibodies which recognize linear epitopes in region 1 were directed against this epitope.

Since human sera cross-reacted with the MBP it was not possible to use the MBP fusion proteins to detect antibodies against region 1. Therefore, the presence of region 1-specific antibodies in human sera was investigated using a blocking ELISA. In this assay the ability of human antibodies to compete with monoclonal antibody PeM7, which binds to region 1 (see above), for binding to immobilized Prn1 was assessed. The serum sample was from a child recently infected with B. pertussis. Similar results were obtained with pooled sera derived from several children (not shown). Binding of PeM7 to Prn1 was inhibited in a dose-dependent manner (Fig. 5), indicating the presence of human antibodies directed against region 1. Binding of PeM2, which recognizes an epitope present in all tested pertactin variants (Table 2) was not inhibited by human antibodies (Fig. 5). The monoclonal antibody PeM5 is similar to Pem7 in that it binds to a conformational epitope encompassing region 1 (see above), also competed with human antibodies (not shown). Interestingly, monoclonal antibodies which recognized linear epitopes (e.g. PeM4 and F6E5) in region 1 were not able to compete with human serum for binding to pertactin (not shown). These results indicate that, in its native state, region 1 elicits antibodies in mice and humans.

### 3D. Peptides derived from pertactin region 1 elicit protective immunity:

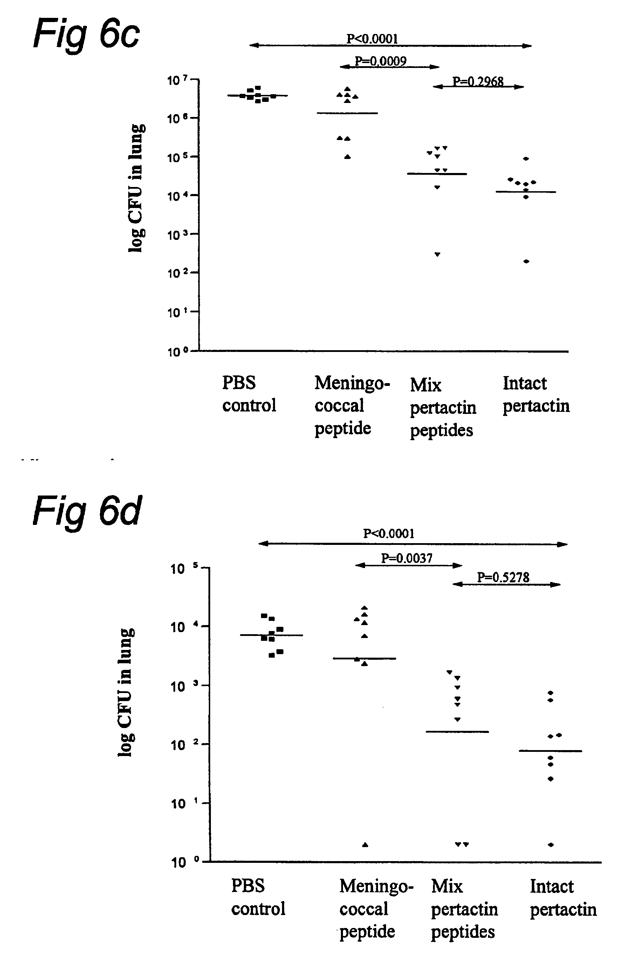

To determine whether region 1 was able to induce a protective immune response, mice were immunized with a mixture of seven overlapping peptides derived from region 1 (pep1 to pep7, Fig. 2a). Mice in the control group were immunized with a meningococcal peptide (Fig. 2a) or with PBS. Immunization with Prn1 served as a positive control. Both pertactin peptides and meningococcal peptides were conjugated to tetanus toxoid. Analysis of the serum samples of immunized and infected mice revealed that high antibody titers to tetanus toxoid were observed in both groups immunized with the peptides (Fig. 6a), while only mice immunized with the pertactin peptides or with the intact pertactin had anti-pertactin titers (Fig. 6b). Mice immunized with the pertactin peptides had up to 35-fold less bacteria in their lungs compared to the group immunized with the meningococcal peptide (P = 0.0009, Fig. 6c). The level of protection induced by the pertactin peptides was 6-fold less than that achieved by intact pertactin (P = 0.2968). Immunization with pertactin peptides reduced colonization in the trachea 16-fold compared to mice immunized with the meningococcal peptide (P = 0.0037, Fig. 6d). As was observed for the lungs, immunization with pertactin peptides was somewhat less (3-fold) effective compared to immunization with native pertactin (P = 0.5278, Fig. 6d). In conclusion, both in the trachea and lungs, peptides derived from region 1 were able to induce protective immunity.

### 3E. Passive immunization with a monoclonal antibody against region 1 confers protection:

It was investigated whether a monoclonal antibody directed against region 1 was also able to protect against infection. PeM4, which binds to the GGFGPGGFGP sequence in region 1 (see above), was administered intravenously 24 h prior to challenge. Control mice were injected with a monoclonal antibody directed against the mumps virus or with PBS. Mice injected with PeM4 showed over 10-fold less colonization of the lungs by B. pertussis, compared to mice injected with PBS (P < 0.0001) (Fig. 7). Passive immunization with the mumps virus monoclonal antibody failed to reduce B. pertussis colonization of the lungs comparable to the PBS control. In the trachea the reduction in colonization by PeM4 was less pronounced, in some experiments an approximately two-fold drop (P = 0.068) in colonization was seen whereas in others no significant drop in colonization was observed (not shown) Taken together, these results confirm that the immune response directed to pertactin region 1 contributes to protection in the lungs.

### 3F. A monoclonal antibody against a conserved linear epitope in region 1 confers cross-protection against strains with different pertactin variants:

Since the monoclonal antibody PeM4 recognizes an epitope present in all known B. pertussis variants of pertactin (Figs 1 and 2b) it was determined whether PeM4 was able to protect against strains which differed in the type of pertactin expressed. Mice were passively immunized with PeM4 24 h before challenge with B. pertussis strain B213 (Prn1), B1296 (Prn2) or B1400 (Prn3). Control mice were injected with PBS before challenge. PeM4 significantly (P ≤ 0.0015) reduced the colonization of B. pertussis stains expressing Prn1, Prn2 and Prn3 in the lungs compared to the PBS controls (Fig. 8), showing that it is possible to elicit antibodies that protect equally against strains expressing distinct pertactin variants.

### 4. Discussion

Studies in mice and men have indicated that pertactin plays an important role in immunity to pertussis. Especially relevant are the results of 2 independent field trials, which revealed a correlation between pertactin antibodies and clinical protection ( Cherry, and Storsaeter; both *supra*). Previous studies have provided indirect, epidemiological, evidence that variation in pertactin is immunologically relevant and may be one of the causes of the re-emergence of pertussis in vaccinated populations (De Melker; Mastrantonio; Mooi et al. (1998) and Mooi et al. (1999), all *supra*). Strains with vaccine-type pertactin were found in lower frequencies in vaccinated children compared to non-vaccinated children, suggesting that variation in pertactin affects vaccine efficacy ( Mooi et al. (1998), *supra*). Further, using a mouse model, it was observed that a WCV with Prn1 is less effective against strains producing non-vaccine type pertactins, compared to strains with Prn1 (results not shown). Here, the immunological relevance of the variation in pertactin is investigated.

The focus was on region 1, since variation is essentially confined to this part of pertactin (Fig. 1). It was observed that a significant part of the mice antibodies were induced against region 1 after immunization with pertactin. Moreover, also in serum from humans recently infected with *B. pertussis,* antibodies against region 1 were detected.

Interestingly, monoclonal antibodies that recognized a conformational epitope in region 1, but not those that bound to a linear epitope, were found to compete with the human antibodies. The reason for this difference is not clear, but it is conceivable that antibodies directed against conformational epitopes bind with higher avidity than antibodies against linear epitopes. It is also possible that infection or vaccination mainly results in the induction of antibodies directed against conformational epitopes of pertactin. In this context it is significant that monoclonal antibodies directed against conformational epitopes were type specific, whereas those that recognized a linear epitope bound to all *B. pertussis* pertactin variants. Thus, to prevent cross-immunity, the bacteria may have evolved mechanisms to direct the immune response towards conformational epitopes. These data show that region 1 is exposed and able to elicit antibodies both in mice and humans. This is consistent with functional studies, which have implicated this region in adherence, and crystallographic data which indicate that region 1 is exposed as a loop (Emsley (1996), *supra*).

Several observations indicated that polymorphism in region 1 affected the binding of antibodies, i.e. represented antigenic variation. Two monoclonal antibodies, presumably directed against conformational epitopes, showed differential binding to region 1 of pertactin variants. Further, polyclonal antiserum raised in mice with pertactin type 1 showed differential binding to the MBP-Prn region 1 fusion proteins.

A number of linear epitopes recognized by monoclonal antibodies were mapped within region 1. One of these monoclonal antibodies (F6E5) was previously shown to inhibit invasion of HeLa cells by *B. pertussis,* and its binding site was mapped to a region of 128 amino acid residues overlapping the RGD site. The F6E5 epitope was defined more precisely with synthetic peptides and located it in the GGxxP repeat region, 23 amino acids removed from the RGD motif. The inhibitory effect of this monoclonal antibody on invasion (Leininger et al., Proc. Natl. Acad. Sci. USA. (1991) 88:345-349) underlines the importance of antigenic variation in region 1 for bacterial fitness.

Immunization of mice with peptides derived from region 1 significantly reduced colonization of both trachea and lungs, demonstrating the importance of this region for immunity. Immunization with a MBP-pertactin fusion protein that contained region 1 did not result in protection, although all mice had high anti-MBP titers (data not shown). However, anti-pertactin titers in these mice were absent or low indicating that the immune response to the pertactin part in the fusion proteins was hampered. The reason for the poor immune response is not known.

Further evidence for the importance of region 1 in protective immunity was revealed by the observation that a monoclonal antibody (PeM4) directed against a linear epitope in region 1, comprising 2 GGFGP repeats, was able to confer passive protection in the lungs. The efficacy of this monoclonal antibody was much lower in the trachea, however. Protection probably requires transudation of antibodies from the blood to the site of infection (i.e. the mucosal surfaces). It is conceivable that the monoclonal antibody used was inefficiently transudated to the trachea. It is not clear on what mechanism the protection by the monoclonal antibody is based. Effector functions, such as opsonisation and complement activation may be involved. The isotype of antibody used, IgG1, shows relatively weak effector functions in the mice (Ravetch and Clynes, Annu. Rev. Immunol. (1998) 16:421-432). Alternatively, the monoclonal antibodies may block the function of pertactin or hinder colonization by agglutinating bacteria. It should be noted that a function for pertactin in the mouse model has yet to be established: a pertactin-defective mutant was not affected in its ability to colonize the trachea or lungs of mice (Roberts et al., Mol. Microbiol. (1991) 5:1393-1404).

A second protective epitope has been located in the PQP repeat region, designated region 2, which is located in the C-terminal part of pertactin (Fig. 1). Like the GGxxP repeat, region 2 was found to be immunodominant in mice. However, the PQP region is less variable than the GGxxP region and of the hundreds of strains analyzed, only 2 polymorphisms were observed in the PQP region (Fig. 1). To date, the minor variant represented by Prn6 was found in 2 isolates only. Thus the vast majority of strains analyzed do not show variation in the PQP region, suggesting that it plays a less important role in escaping immunity compared to region 1.

Monoclonal antibody PeM4, which recognized an epitope in region 1 that is present in all *Bordetella pertussis* pertactin types found to date, conferred passive protection against *B. pertussis* strains carrying different pertactin variants (Fig. 8). Further, a peptide (GGAVPGGFGPGGFGP) found in all pertactin types was protective in the mouse model These observations raise the exciting possibility of a vaccine that is equally efficacious against all *B. pertussis* pertactin variants.

From the data described hereinabove it can be concluded that the polymorphic region 1 of pertactin is recognized by the immune system of both mice and humans, that variation in this region affects antibody binding and that this region harbors a protective epitope. These results are consistent with the assumption that the expansion of strains which carry non-vaccine type pertactin molecules has contributed to the re-emergence of pertussis. Importantly, it is shown that it is possible to elicit pertactin antibodies that protect against strains with distinct pertactin variants, providing a basis for further improvement of pertussis vaccines.

### Example 2: Immunisation of mice with the peptide GGAVP-GGFGP-GGFGP

The sequence GGAVP-GGFGP-GGFGP is found in region of all pertactin variants identified to date. Mice were immunized with this peptide to determine if it conferred protection against *B.pertussis*. As indicated in the figure, mice immunized with GGAVP-GGFGP-GGFGP were significantly less colonized in the lungs compared to mice vaccinated with a control (meningoccal) peptide (P = 0.0027). A second peptide GGFGP-GGFGP-GGFGP) found in region 1 of the 2 predominant pertactin types in The Netherlands, Finland and Italy (i.e. pertactin type 2 and 3) also conferred significant protection compared to the control peptide (P = 0.0023). These results provide addtional evidence that conserved sequences from region 1 can induce cross-protective antibodies.

**Table 1.**

| *Bordetella* strains used in this application. | | | | | |
|---|---|---|---|---|---|
| **Strain** | **original designation** | **species** | **Source** | **prn type** | **Accession number** |
| B5^{a} | | *B.pertussis* | US | *prn1* | AJ011091 |
| B391^{b} | | *B.pertussis* | Netherlands | *prn1* | AJ011091 |
| B394^{a} | | *B.pertussis* | Netherlands | *prn2* | AJ011092 |
| B596^{b,a} | | *B.pertussis* | Netherlands | *prn2* | AJ011092 |
| B365^{b} | | *B.pertussis* | Netherlands | *prn3* | AJ011093 |
| B647^{b} | | *B.pertussis* | Netherlands | *prn3* | AJ011093 |
| B705^{b,a} | | *B.pertussis* | Finland | *prn4* | AJ011015 |
| B935^{b} | | *B.pertussis* | Italy | *prn5* | AJ011016 |
| B1148^{a} | | *B.pertussis* | Italy | *prn5* | AJ011016 |
| B1120^{b} | 18323 | *B.pertussis* | US | *prn6* | AJ132095 |
| B213^{c} | Tohama | *B.pertussis* | Japan | *prn1* | AJ011091 |
| B1296^{c} | | *B.pertussis* | Netherlands | *prn2* | AJ011092 |
| B1400^{c} | | *B.pertussis* | Netherlands | *prn3* | AJ011093 |
| B14^{b} | | *B.bronchiseptica* | | *prn* | AJ245927 |
| B24^{b} | | *B.parapertussis* | Netherlands | *prn* | X54547 |

| | | | | | |
|---|---|---|---|---|---|
| ^{a} Used for construction of the MBP-fusion proteins | | | | | |
| ^{b} Used for immunoblotting. | | | | | |
| ^{c} Used for challenge experiments. ^{d} Accession number refers to an identical, previously submitted, sequence from another strain. | | | | | |

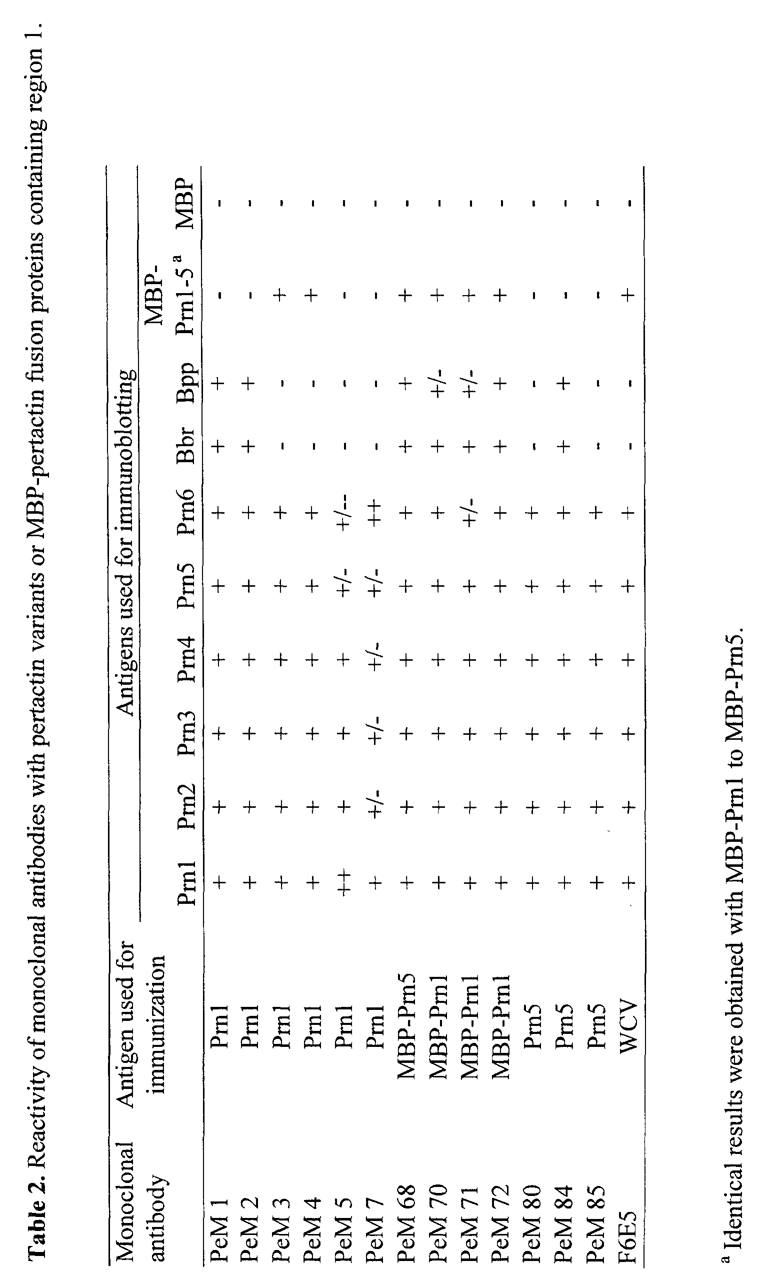

## Claims

1. Peptide, comprising:
c) between 6 and 40 contiguous amino acid residues from the amino acid sequence of "region 1" of a *Bordetella pertussis* pertactin; and
d) a total of between 6 and 100, preferably between 6 and 50 amino acid residues;
or an analog of such a peptide; optionally in the form of a salt acceptable for pharmaceutical and/or veterinary purposes.

2. Peptide according to claim 1, comprising:
a) between 10 and 15 contiguous amino acid residues from the amino acid sequence of "region 1" of a *Bordetella pertussis* pertactin; and
b) a total of between 10 and 40 amino acid residues.
or an analog of such a peptide; optionally in the form of a salt acceptable for pharmaceutical and/or veterinary purposes.

3. Peptide according to claim 1 and/or 2, comprising
a) between 6 and 36 contiguous amino acid residues from the amino acid sequence of SEQ ID NO:1 TIRRGDAPAGGAVPGGAVPGGAVPGGFGPGGFGPVL (SEQ ID NO:1) and
b) a total of between 6 and 100, preferably between 6 and 50 amino acid residues;
or an analog thereof; optionally in the form of a salt acceptable for pharmaceutical and/or veterinary purposes.

4. Peptide according to claim 3, comprising
a) between 10 and 15 contiguous amino acid residues from the amino acid sequence of SEQ ID NO:1 TIRRGDAPAGGAVPGGAVPGGAVPGGFGPGGFGPVL (SEQ ID NO:1) and
b) a total of between 10 and 40 amino acid residues;
or an analog thereof; optionally in the form of a salt acceptable for pharmaceutical and/or veterinary purposes.

5. Peptide according to any of the preceding claims, comprising one or more repeats GGFGP and/or one or more repeats GGAVP, in which the total number of such repeats is between 2 and 10; or an analog thereof; optionally in the form of a salt acceptable for pharmaceutical and/or veterinary purposes.

6. Peptide according to any of the preceding claims, which provides at least 10%, preferably at least 30%, more preferably at least 50%, and even more preferably at least 70%, of the protection against *Bordetella pertussis,* and preferably also against *Bordetella parapertussis*, provided by the peptide of SEQ ID NO:5 GGAVPGGFGPGGFGP (SEQ ID NO:5) or an analog thereof; optionally in the form of a salt acceptable for pharmaceutical and/or veterinary purposes.

7. Peptide according to any of the preceding claims, having the amino acid sequence of SEQ ID NO:5: GGAVPGGFGPGGFGP (SEQ ID NO:5) or an analog thereof; optionally in the form of a salt acceptable for pharmaceutical and/or veterinary purposes.

8. Pharmaceutical composition, and in particular vaccine, comprising at least one peptide according to any of the preceding claims, optionally in combination with:
a) at least one pharmaceutically acceptable carrier, excipiens and/or carrier; and/or
b) one or more immunological adjuvantia; and/or
c) one or more further (para)pertussis antigens; and/or
d) one or more further pharmaceutically acceptable components that, upon administration to a human being, can protect said human being against one or more infectious diseases other than pertussis.

9. Veterinary composition, and in particular vaccine, comprising at least one peptide according to any of the preceding claims, optionally in combination with:
a) at least one carrier, excipiens and/or carrier acceptable for veterinary purposes; and/or
b) one or more immunological adjuvantia; and/or
c) one or more further (para)perlussis antigens; and/or
d) one or more further components acceptable for veterinary purposes that, upon administration to a mammal, can protect said mammal against one or more infectious diseases of said mammal other than pertussis.

10. Use of a peptide according to any of claims 1-8 in the preparation of a vaccine against *Bordetella pertussis,* and preferably also against *Bordetella parapertussis*, and optionally also against one or more further infectious diseases of a human being other than parapertussis.

11. Use of a peptide according to any of claims 1-8 in the preparation of a veterinary vaccine against *Bordetella pertussis,* and preferably also against *Bordetella parapertussis and/or Bordetella brochiseptica*, and optionally also against one or more further infectious disease of a mammal other than parapertussis.

12. Protein fusion, comprising at least one peptide according to claims 1-8 and at least one further amino acid sequence which is different from the amino acid sequence with which a peptide of the invention is (or may be) natively associated in a naturally occuring pertactin.

13. Pharmaceutical composition, and in particular vaccine, comprising at least one fusion according to claim 12, optionally in combination with:
a) at least one pharmaceutically acceptable carrier, excipiens and/or carrier; and/or
b) one or more immunological adjuvantia; and/or
c) one or more further (para)pertussis antigens; and/or
d) one or more further pharmaceutically acceptable components that, upon administration to a human being, can protect said human being against one or more infectious diseases other than pertussis.

14. Veterinary composition, and in particular vaccine, comprising at least one protein fusion according claim 12, optionally in combination with:
a) at least one carrier, excipiens and/or carrier acceptable for veterinary purposes; and/or
b) one or more immunological adjuvantia; and/or
c) one or more further (para)pertussis antigens; and/or
d) one or more further components acceptable for veterinary purposes that, upon administration to a mammal, can protect said mammal against one or more infectious diseases of said mammal other than pertussis.

15. Polypeptide having the amino acid sequence of a pertactin from which, compared to the sequence of said pertactin:
a) the amino acid residues that form the so-called "region 1" have been at least partly, and preferably essentially completely removed; and/or from which,
b) the amino acid residues that form the so-called "region 2" have been at least partly, and preferably essentially completely removed.

16. Polypeptide according to claim 15, having the amino acid sequence of pertactin as shown in SEQ ID NO:6 - or the amino acid sequence of a naturally occurring or synthetic analog or variant thereof - from which:
a) at least 5, preferably at least 10, and up to all of the amino acid residues at positions 266 - 290 have been removed; and/or from which
b) at least 3, preferably at least 10, and up to all of the amino acid residues at positions 569-603 have been removed.

17. Polypeptide according to claim 14 or 15, having the amino acid sequence of pertactin as shown in SEQ ID NO:6 - or the amino acid sequence of a naturally occurring or synthetic analog or variant thereof - from which one or more amino acid residues are/have been removed (i.e. as outlined above) such that
a) from the amino acid residues between positions 260 and 290 of SEQ ID NO:6, at least one repeat "GGAVP" and/or at least one repeat "GGFGP" is/has been removed, and up to all such repeats "GGAVP" and/or "GGFGP" that are present between positions 260 and 290; and/or such that.
b) from the amino acid residues between positions 569 and 603 of SEQ ID NO:6, at least one motif "PQP" is/has been removed, and up to all motifs "PQP" that are present between positions 569 and 603.

18. Polypeptide according to any of claims 15-17, having the amino acid sequence of pertactin as shown in SEQ ID NO:6 - or the amino acid sequence of a naturally occurring or synthetic analog and/or variant thereof - from which the following amino acid residues have been removed:
a) the residues from Gly at position 266 up to and including Pro at position 290; or
b) the residues from Gly at position 270 up to and including Pro at position 285; and/or from which any of the following amino acid residues have been removed.
a) the residues from Ala at position 569 up to and including Ala at position 603; or
b) the residues from Pro at position 572 up to and including Gln at position 599; or
c) the residues from Pro at position 575 up to and including Pro at position 596; or
d) the residues from Ala at position 578 up to and including Glu at position 594; or
e) the residues from Pro at position 581 up to and including Pro at position 591; or
f) the residues from Gln at position 584 up to and including Pro at position 588.

19. Pharmaceutical composition, and in particular vaccine, comprising at least one polypeptide according to any of claims 14-18, optionally in combination with:
a) at least one pharmaceutically acceptable carrier, excipiens and/or carrier; and/or
b) one or more immunological adjuvantia; and/or
c) one or more further (para)pertussis antigens; and/or
d) one or more further pharmaceutically acceptable components that, upon administration to a human being, can protect said human being against one or more infectious diseases other than pertussis.

20. Veterinary composition, and in particular vaccine, comprising at least one polypeptide according to any of claims 14-18, optionally in combination with:
a) at least one carrier, excipiens and/or carrier acceptable for veterinary purposes; and/or
b) one or more immunological adjuvantia; and/or
c) one or more further (para)pertussis antigens; and/or
d) one or more further components acceptable for veterinary purposes that, upon administration to a mammal, can protect said mammal against one or more infectious diseases of said mammal other than pertussis.

21. Use of a polypeptide according to any of claims 14-18 in the preparation of a vaccine against *Bordetella pertussis,* and preferably also against *Bordetella parapertussis*, and optionally also against one or more further infectious diseases of a human being other than parapertussis.

22. Use of a polypeptide according to any of claims 14-18 in the preparation of a veterinary vaccine against *Bordetella pertussis,* and preferably also against *Bordetella parapertussis and/or Bordetella brochiseptica*, and optionally also against one or more further infectious disease of a mammal other than parapertussis.

23. Antibody, generated/elicited against a peptide according to any of claims 1-8 and/or against a polypeptide according to any of claims 14-18.

24. Pharmaceutical and/or veterinary composition comprising at least one antibody according to claim 23, and optionally at least one carrier, excipiens and/or adjuvans acceptable for pharmaceutical and/or veterinary purposes.


SEQUENCE LISTING

<110> Staat der Nederlanden

<120> Peptides for the preparation of vaccines against
      Bordetella pertussis and/or Bordetella parapertussis,
      vaccines based upon such peptides, and antibodies
      against such peptides.

<130> BO 43642

<140> EP
<141> 2000-06-30

<160> 8

<170> PatentIn Ver. 2.1

<210> 1
<211> 36
<212> PRT
<213> Bordetella pertussis

<400> 1
Thr Ile Arg Arg Gly Asp Ala Pro Ala Gly Gly Ala Val Pro Gly Gly
  1               5                  10                  15

Ala Val Pro Gly Gly Ala Val Pro Gly Gly Phe Gly Pro Gly Gly Phe
             20                  25                  30

Gly Pro Val Leu
         35

<210> 2
<211> 10
<212> PRT
<213> Artificial Sequence

<220>

<223> Description of Artificial Sequence: peptide

<400> 2
Gly Gly Phe Gly Pro Gly Gly Phe Gly Pro
1               5                   10

<210> 3
<211> 10
<212> PRT
<213> Artificial Sequence

<220>
<223> Description of Artificial Sequence: peptide

<400> 3
Gly Gly Ala Val Pro Gly Gly Ala Val Pro
1               5                   10

<210> 4
<211> 20
<212> PRT
<213> Artificial Sequence

<220>
<223> Description of Artificial Sequence: peptide

<400> 4
Gly Gly Ala Val Pro Gly Gly Ala Val Pro Gly Gly Phe Gly Pro Gly
1               5                   10                  15

Gly Phe Gly Pro
            20

<210> 5
<211> 15

<212> PRT
<213> Artificial Sequence

<220>
<223> Description of Artificial Sequence: peptide

<400> 5
Gly Gly Ala Val Pro Gly Gly Phe Gly Pro Gly Gly Phe Gly Pro
1               5                   10                  15

<210> 6
<211> 910
<212> PRT
<213> Bordetella pertussis

<400> 6
Met Asn Met Ser Leu Ser Arg Ile Val Lys Ala Ala Pro Leu Arg Arg
1               5                   10                  15

Thr Thr Leu Ala Met Ala Leu Gly Ala Leu Gly Ala Ala Pro Ala Ala
            20                  25                  30

His Ala Asp Trp Asn Asn Gln Ser Ile Val Lys Thr Gly Glu Arg Gln
        35                  40                  45

His Gly Ile His Ile Gln Gly Ser Asp Pro Gly Gly Val Arg Thr Ala
    50                  55                  60

Ser Gly Thr Thr Ile Lys Val Ser Gly Arg Gln Ala Gln Gly Ile Leu
65                  70                  75                  80

Leu Glu Asn Pro Ala Ala Glu Leu Gln Phe Arg Asn Gly Ser Val Thr
                85                  90                  95

Ser Ser Gly Gln Leu Ser Asp Asp Gly Ile Arg Arg Phe Leu Gly Thr
            100                 105                 110

Val Thr Val Lys Ala Gly Lys Leu Val Ala Asp His Ala Thr Leu Ala
        115                 120                 125

Asn Val Gly Asp Thr Trp Asp Asp Asp Gly Ile Ala Leu Tyr Val Ala
    130                 135                 140

Gly Glu Gln Ala Gln Ala Ser Ile Ala Asp Ser Thr Leu Gln Gly Ala
145                 150                 155                 160

Gly Gly Val Gln Ile Glu Arg Gly Ala Asn Val Thr Val Gln Arg Ser
                165                 170                 175

Ala Ile Val Asp Gly Gly Leu His Ile Gly Ala Leu Gln Ser Leu Gln
            180                 185                 190

Pro Glu Asp Leu Pro Pro Ser Arg Val Val Leu Arg Asp Thr Asn Val
        195                 200                 205

Thr Ala Val Pro Ala Ser Gly Ala Pro Ala Ala Val Ser Val Leu Gly
    210                 215                 220

Ala Ser Glu Leu Thr Leu Asp Gly Gly His Ile Thr Gly Gly Arg Ala
225                 230                 235                 240

Ala Gly Val Ala Ala Met Gln Gly Ala Val Val His Leu Gln Arg Ala
                245                 250                 255

Thr Ile Arg Arg Gly Asp Ala Pro Ala Gly Gly Ala Val Pro Gly Gly
            260                 265                 270

Ala Val Pro Gly Gly Ala Val Pro Gly Gly Phe Gly Pro Gly Gly Phe
        275                 280                 285

Gly Pro Val Leu Asp Gly Trp Tyr Gly Val Asp Val Ser Gly Ser Ser
    290                 295                 300

Val Glu Leu Ala Gln Ser Ile Val Glu Ala Pro Glu Leu Gly Ala Ala
305                 310                 315                 320

Ile Arg Val Gly Arg Gly Ala Arg Val Thr Val Ser Gly Gly Ser Leu
                325                 330                 335

Ser Ala Pro His Gly Asn Val Ile Glu Thr Gly Gly Ala Arg Arg Phe

            340                 345                 350

Ala Pro Gln Ala Ala Pro Leu Ser Ile Thr Leu Gln Ala Gly Ala His
        355                 360                 365

Ala Gln Gly Lys Ala Leu Leu Tyr Arg Val Leu Pro Glu Pro Val Lys
    370                 375                 380

Leu Thr Leu Thr Gly Gly Ala Asp Ala Gln Gly Asp Ile Val Ala Thr
385                 390                 395                 400

Glu Leu Pro Ser Ile Pro Gly Thr Ser Ile Gly Pro Leu Asp Val Ala
                405                 410                 415

Leu Ala Ser Gln Ala Arg Trp Thr Gly Ala Thr Arg Ala Val Asp Ser
            420                 425                 430

Leu Ser Ile Asp Asn Ala Thr Trp Val Met Thr Asp Asn Ser Asn Val
        435                 440                 445

Gly Ala Leu Arg Leu Ala Ser Asp Gly Ser Val Asp Phe Gln Gln Pro
    450                 455                 460

Ala Glu Ala Gly Arg Phe Lys Val Leu Thr Val Asn Thr Leu Ala Gly
465                 470                 475                 480

Ser Gly Leu Phe Arg Met Asn Val Phe Ala Asp Leu Gly Leu Ser Asp
                485                 490                 495

Lys Leu Val Val Met Gln Asp Ala Ser Gly Gln His Arg Leu Trp Val
            500                 505                 510

Arg Asn Ser Gly Ser Glu Pro Ala Ser Ala Asn Thr Leu Leu Leu Val
        515                 520                 525

Gln Thr Pro Leu Gly Ser Ala Ala Thr Phe Thr Leu Ala Asn Lys Asp
    530                 535                 540

Gly Lys Val Asp Ile Gly Thr Tyr Arg Tyr Arg Leu Ala Ala Asn Gly
545                 550                 555                 560

Asn Gly Gln Trp Ser Leu Val Gly Ala Lys Ala Pro Pro Ala Pro Lys
                565                 570                 575

Pro Ala Pro Gln Pro Gly Pro Gln Pro Pro Gln Pro Pro Gln Pro Gln
            580                 585                 590

Pro Glu Ala Pro Ala Pro Gln Pro Pro Ala Gly Arg Glu Leu Ser Ala
        595                 600                 605

Ala Ala Asn Ala Ala Val Asn Thr Gly Gly Val Gly Leu Ala Ser Thr
    610                 615                 620

Leu Trp Tyr Ala Glu Ser Asn Ala Leu Ser Lys Arg Leu Gly Glu Leu
625                 630                 635                 640

Arg Leu Asn Pro Asp Ala Gly Gly Ala Trp Gly Arg Gly Phe Ala Gln
                645                 650                 655

Arg Gln Gln Leu Asp Asn Arg Ala Gly Arg Arg Phe Asp Gln Lys Val
            660                 665                 670

Ala Gly Phe Glu Leu Gly Ala Asp His Ala Val Ala Val Ala Gly Gly
        675                 680                 685

Arg Trp His Leu Gly Gly Leu Ala Gly Tyr Thr Arg Gly Asp Arg Gly
    690                 695                 700

Phe Thr Gly Asp Gly Gly Gly His Thr Asp Ser Val His Val Gly Gly
705                 710                 715                 720

Tyr Ala Thr Tyr Ile Ala Asp Ser Gly Phe Tyr Leu Asp Ala Thr Leu
                725                 730                 735

Arg Ala Ser Arg Leu Glu Asn Asp Phe Lys Val Ala Gly Ser Asp Gly
            740                 745                 750

Tyr Ala Val Lys Gly Lys Tyr Arg Thr His Gly Val Gly Ala Ser Leu
        755                 760                 765

Glu Ala Gly Arg Arg Phe Thr His Ala Asp Gly Trp Phe Leu Glu Pro
    770                 775                 780

Gln Ala Glu Leu Ala Val Phe Arg Ala Gly Gly Gly Ala Tyr Arg Ala
785                 790                 795                 800

Ala Asn Gly Leu Arg Val Arg Asp Glu Gly Gly Ser Ser Val Leu Gly
                805                 810                 815

Arg Leu Gly Leu Glu Val Gly Lys Arg Ile Glu Leu Ala Gly Gly Arg
            820                 825                 830

Gln Val Gln Pro Tyr Ile Lys Ala Ser Val Leu Gln Glu Phe Asp Gly
        835                 840                 845

Ala Gly Thr Val His Thr Asn Gly Ile Ala His Arg Thr Glu Leu Arg
    850                 855                 860

Gly Thr Arg Ala Glu Leu Gly Leu Gly Met Ala Ala Ala Leu Gly Arg
865                 870                 875                 880

Gly His Ser Leu Tyr Ala Ser Tyr Glu Tyr Ser Lys Gly Pro Lys Leu
                885                 890                 895

Ala Met Pro Trp Thr Phe His Ala Gly Tyr Arg Tyr Ser Trp
            900                 905                 910

<210> 7
<211> 26
<212> DNA
<213> Artificial Sequence

<220>
<223> Description of Artificial Sequence: primer

<400> 7
cgggatccac gatacggcgc ggggac                                          26

<210> 8
<211> 26

<212> DNA
<213> Artificial Sequence

<220>
<223> Description of Artificial Sequence: primer

<400> 8
gctctagaga ggacgggacc gaagcc                                          26